# EUROPEAN PATENT APPLICATION

(11) **EP 1 897 870 A1**
(43) Date of publication of application: **12.03.2008**
(21) Application number: 06767680.9
(22) Date of filing: 30.06.2006
(51) Int. Cl.: C07D 213/70, A61K 31/4412, A61K 31/4439, A61K 31/497, A61K 31/506, A61P 1/04, A61P 3/10, A61P 9/10, A61P 11/00, A61P 11/06, A61P 17/00, A61P 17/06, A61P 19/02, A61P 19/10, A61P 25/00, A61P 29/00, A61P 35/04, A61P 37/02, A61P 37/06, A61P 37/08, A61P 43/00

(54) **PHARMACEUTICAL COMPOSITION COMPRISING 2-PYRIDONE DERIVATIVE AS ACTIVE INGREDIENT**

(30) Priority: 30.06.2005 JP 2005192821
(71) Applicant: Sato Pharmaceutical Co. Ltd., Tokyo 107-0051 (JP)
(72) Inventor: KATO, Masatoshi c/o R & D Center, SATO Pharmaceuti, ku, Tokyo;1400011 (JP); KUSAKABE, Hiroyuki c/o R & D Center, SATO Pharmace, wa-ku, Tokyo;1400011 (JP); YANAGIHARA, Satoshi c/o R & D Center, SATO Pharmac, awa-ku, Tokyo;400011 (JP); AKIZAWA, Hidenori c/o R & D Center, SATO Pharmaceu, a-ku, Tokyo;1400011 (JP); TAMOTO, Yuji c/o R & D Center, SATO Pharmaceutical, Tokyo;1400011 (JP)
(74) Representative: Hengelhaupt, Jürgen
(86) International application number: PCT/JP2006/313072
(87) International publication number: WO 2007/004543

(57) **Abstract**

The present invention herein provides a 2-pyridone derivative which has a cell adhesion-inhibitory activity and which is useful for preventing and/or treating pathema or disease conditions including, for instance, inflammations which relate to leukocytic infiltration. The 2-pyridone derivative is a compound represented by the following general formula (1). In the formula, R¹ represents, for instance, a phenyl or pyridyl group, R² represents, for instance, a phenyl group, R³ represents, for instance, a lower alkyl group, R⁴ represents, for instance, a lower alkyl group, R⁵ represents, for instance, a lower alkyl group, and R⁶ represents, for instance, a lower alkyl group:

## Description

### Technical Field

The present invention relates to a novel compound having a strong cell adhesion-inhibitory activity and more specifically a novel compound useful for preventing and/or treating disease conditions, including inflammations, allergies, rheumatisms, cancer metastasis, asthmas, diabetes and autoimmune diseases, which are associated with leukocytic infiltration due to the action of, for instance, neutrophils, as well as a medicine containing the compound as an effective component.

### Background Art

There have conventionally and widely been used NSAID (non-steroidal anti-inflammatory agents) and COX-II inhibitory agents in the treatment of diseases such as inflammations which are caused due to the leukocytic infiltration. However, chemical mediators such as cytokines in the arachidonic acid cascade have various kinds of physiological activities and accordingly, a problem arises in that, if they are suppressed, side-effects may take place. Therefore, the amount of a medicine to be administered should be limited to a desired level and it would thus be necessary to take into consideration the balance between the efficacy and the side-effects thereof.

Diseases accompanied by inflammation including a variety of allergies are caused by the damages of tissues at inflamed sites due to any excess immune reaction. At this stage, it has been believed that the following mechanism or the cell adhesion is a quite important step: the migratory immunocytes such as leukocytes are activated through the expression and display of adhesive molecules, they are then adhered to the vascular endothelial cells in the proximity to the inflamed sites and they infiltrate into the extravascular tissues (Cell, 65, 859-873, 1991; Cell, 67, 1033-1036, 1991; Immunol. Today, 14, 99-102, 1993; Cell, 76, 301-314, 1994). Moreover, in addition to the immune reaction, the cell adhesion would greatly participate in phenomena such as the metastasis of cancer.

The medicine, which acts on the cell adhesion molecule as a target, directly functions in the middle (or midstream) of the disease state-generation mechanism in which the medicine may act on the cell adhesion molecule in its activated condition and accordingly, the medicine is considered to provide much less side-effect. Thus, such a medicine would be able to be administered over a long period of time.

For this reason, recently, there have widely been conducted many researches with the aim of controlling the cell adhesion. In particular, the neutrophils first play a central role in the advance of the inflammation (N. Engl. J. Med., 317(11), 687-694, 1987; Ann. Intern. Med., 109, 127-142, 1988; N. Engl. J. Med., 320(6), 365-376, 1989; the Course of Medical Science, 191(2), 143-147, 1999) and accordingly, there has gradually been elucidated the interactions between LFA-1 and Mac-1 as the adhesion molecules thereof and ICAM-1 as the ligand on the vascular endothelial cells (Pathol. Biol., 46, 164-170, 1998) and the relationship thereof with acute inflammation has, in fact, been confirmed (J. Immunol., 150, 655-663, 1993). In addition, the interaction between the LFA-1 and ICAM-1 may play an important role in the antigen-recognition between antigen-presenting cells and T lymphocytes and therefore, the control of any rejection and the extension of the life of a graft in the transplantation have been demonstrated by the studies which make use of a monoclonal antibody in the mouse heart transplantation model (Science, 255, 1125-1127, 1992). Moreover, in respect of the chronic rheumatoid arthritis, there has been reported, for instance, an inflammation-inhibitory effect through the blocking of the LFA-1/ICAM-1 path in the rat's adjuvant arthritis model (J. Immunol., 147, 4167-4171, 1991). In addition to the foregoing, it has been confirmed that the monoclonal antibody against LFA-1/ICAM-1 would be efficacious against various kinds of disease models such as arteriosclerosis (N. Engl. J. Med., 340, 115-126, 1999), the acute respiratory distress syndrome (ADRS)-like acute lung injury (ALI) models (J. Immunol., 150, 2407-2417, 1993) and the non-occlusive mesenteric infarction (Am. J. Pathol., 143, 410-418, 1993; Neurology, 44, 1747-1751, 1994).

However, the antibody often provides problems, for example, in that the antigenicity thereof and the absorptivity thereof through the oral route are low and accordingly, there have been conducted various studies to develop a cell adhesion-inhibitory agent which can act on low molecular compounds as targets, but most of them have still been in course of development.
Heretofore, there have been reported, for instance, cell adhesion-inhibitory agents which act via the LFA-1/ICAM-1 path such as Mevinolin and homologues thereof (see Patent Document 1); diaryl sulfide derivatives (see Patent Document 2); cinnamic acid amide compounds (see Patent Document 3); diazabicyclo-alkanedione derivatives (see Patent Document 4); imidazolidine-dione derivatives (see Patent Document 5); substituted diazepams (see Patent Document 6); diaza-cycloalkane-dione derivatives (see Patent Document 7); hydantoin compounds (see Patent Document 8); phenylalanine derivatives (see Patent Document 9); pyridazinone derivatives (see Patent Document 10); and amino acid derivatives (see, Bioorg.&Med. Chem. Lett., 13, 1015, 2003; Non-Patent Document 1) and other substances (see Patent Documents 11, 12). In addition, there have also been reported compounds each carrying a 2-pyridone skeleton, which possess an anti-inflammatory effect (see Patent Document 13).

However, the foregoing technical documents never disclose and teach such a compound, proposed herein, which has a 2-pyridone skeleton carrying, at a specific site, a sulfide or substituted sulfide-containing group and which shows a cell adhesion-inhibitory effect and a leukocytic infiltration-inhibitory activity.
Patent Document 1: JP-A-2001-514221;
Patent Document 2: JP-A- 2002-541141;
Patent Document 3: JP-A-2002-533434;
Patent Document 4: JP-A-2003-2834;
Patent Document 5: JP-A-2003-535087;
Patent Document 6: JP-A- 2003-51144;
Patent Document 7: JP-A- 2004-510768;
Patent Document 8: JP-A-2004-519435;
Patent Document 9: WO 2002/018320;
Patent Document 10: WO 2000-09488;
Patent Document 11: JP-A-2005-2116;
Patent Document 12: JP-A-2004-502672;
Patent Document 13: U.K. Patent No. 1,238,959.

### Disclosure of the Present Invention

### Problems That the Invention is to Solve

It is an object of the present invention to provide a pharmaceutical composition containing, as an effective component, a novel compound which has a strong cell adhesion-inhibitory activity and which is useful for preventing and/or treating disease conditions including inflammations associated with leukocytic infiltration.

### Means for Solving the Problems

The inventors of this invention have conducted intensive studies to accomplish the foregoing object, have found that 2-pyridone derivatives each having a specific structure have strong cell adhesion-inhibitory activities and are useful as a medicine for preventing and/or treating disease conditions associated with the leukocytic infiltration. The present invention has been completed based on the above finding.

Accordingly, the present invention herein provides the inventions specified below:
1. A 2-pyridone derivative represented by the following formula (1) or a pharmaceutically acceptable salt thereof: 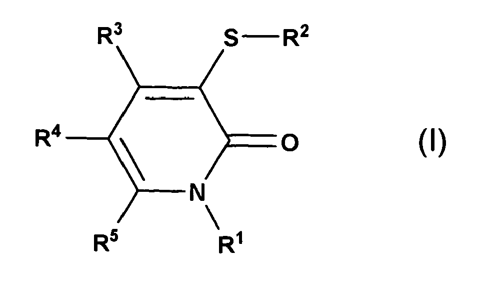 wherein, R¹ represents a hydrogen atom, a lower alkyl group, a cycloalkyl group, an aryl group or an aromatic heterocyclic group;
   R² represents a hydrogen atom, a halogen atom, a cyano group, a lower alkyl group, an aryl group or an aromatic heterocyclic group;
   R³ represents a lower alkyl group or an aryl group;
   R⁴ represents a lower alkyl group, an aryl group, -C(=O)-R⁶, or -CH(OH)-R⁶ (wherein R⁶ represents a lower alkyl group or an aryl group); and
   R⁵ represents a hydrogen atom, a lower alkyl group or an aryl group.
2. A pharmaceutical composition comprising, as an effective component, a 2-pyridone derivative or a pharmaceutically acceptable prodrug or salt thereof as set forth in foregoing item 1.
3. A method for preparing a 2-pyridone derivative represented by the following formula (1): 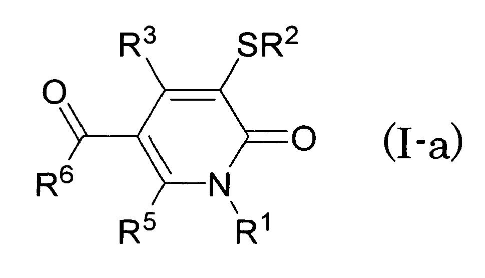 wherein, R¹ represents a hydrogen atom, a lower alkyl group, a cycloalkyl group, an aryl group or an aromatic heterocyclic group;
   R² represents a hydrogen atom, a halogen atom, a cyano group, a lower alkyl group, an aryl group or an aromatic heterocyclic group;
   R³ represents a lower alkyl group or an aryl group;
   R⁵ represents a hydrogen atom, a lower alkyl group or an aryl group; and
   R⁶ represents a lower alkyl group or an aryl group,
   said method comprising reacting a compound (IV) represented by the following formula (IV): 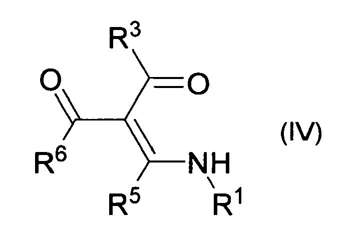 wherein R¹, R³, R⁵, and R⁶ are the same as those specified above) with a compound (V) represented by the following formula (V): 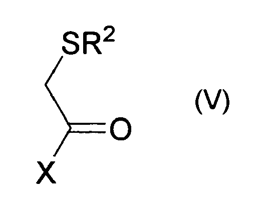 wherein R² is the same as that specified above, and X represents a halogen atom.
4. A method for preparing a 2-pyridone derivative represented by the following formula (1): 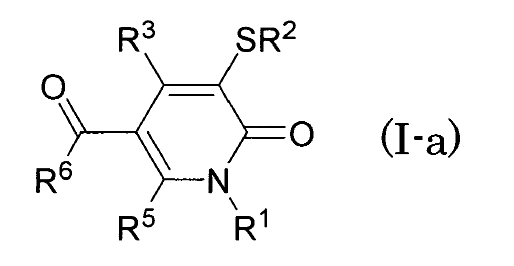 wherein R¹ represents a hydrogen atom, a lower alkyl group, a cycloalkyl group, an aryl group or an aromatic heterocyclic group;
   R² represents a hydrogen atom, a halogen atom, a cyano group, a lower alkyl group, an aryl group or an aromatic heterocyclic group;
   R³ represents a lower alkyl group or an aryl group;
   R⁵ represents a hydrogen atom, a lower alkyl group or an aryl group); and
   R⁶ represents a lower alkyl group or an aryl group,
   said method comprising the steps of:
   (1) reacting a compound (IV) represented by the following formula (IV): 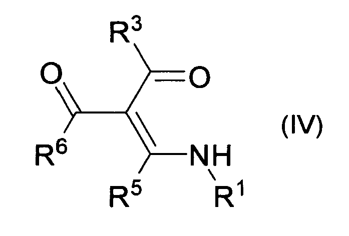 wherein R¹, R³, R⁵, and R⁶ are the same as those specified above, with a compound (VI) represented by the following formula (VI): 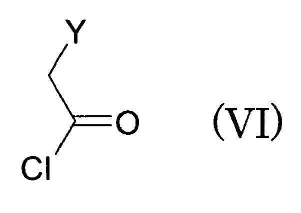 wherein Y represents a halogen atom, to thus form a compound (I-b) represented by the following formula (I-b) : 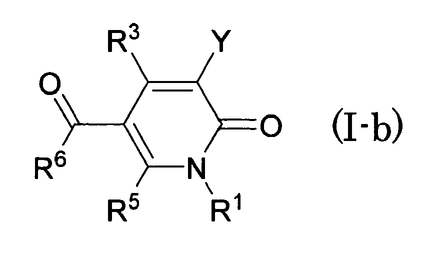 wherein R¹, R³, R⁵, R⁶ and Y are the same as those specified above); and then
   (2) reacting the resulting compound (I-b) with HSR² wherein R² is the same as that specified above.
5. A method for preparing a compound represented by the following formula (1): 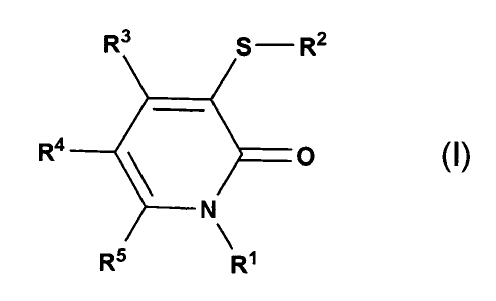 wherein R¹ represents a hydrogen atom, a lower alkyl group, a cycloalkyl group, an aryl group or an aromatic heterocyclic group;
   R² represents a hydrogen atom, a halogen atom, a cyano group, a lower alkyl group, an aryl group or an aromatic heterocyclic group;
   R³ represents a lower alkyl group or an aryl group;
   R⁴ represents a lower alkyl group, an aryl group, -C(=O)-R⁶, or -CH(OH)-R⁶ (wherein R⁶ represents a lower alkyl group or an aryl group); and
   R⁵ represents a hydrogen atom, a lower alkyl group or an aryl group,
   said method comprising the steps of:
   (1) preparing a compound (XII) represented by the following formula (XII): 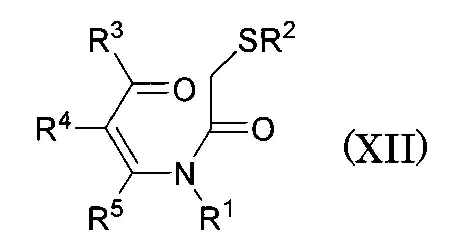 wherein R¹ to R⁵ are the same as those specified above, and then
   (2) treating the resulting compound (XII) with a base.

### Preferred Embodiments of the Invention

The present invention will be described in more detail below.

The 2-pyridone derivative of the present invention is a compound represented by the foregoing formula (1). In the formula, R¹ represents a hydrogen atom, a lower alkyl group, a cycloalkyl group, an aryl group or an aromatic heterocyclic group.
The term "lower alkyl group" appearing in the definition of the substituent R¹ means an alkyl group having 1 to 8 carbon atoms and preferably one having 1 to 5 carbon atoms. Specific examples thereof suitably used herein include methyl, ethyl, propyl, butyl, pentyl, hexyl, heptyl and octyl groups. These lower alkyl groups may be linear or branched ones and further, they may have any substituent. Such substituents suitably used herein may be, for instance, halogen atoms (such as fluorine, chlorine, bromine and iodine atoms), and hydroxyl, phenyl, lower alkoxy, phenyloxy, carboxyl, nitro and amino groups. In this respect, examples of the alkyl moiety of the lower alkoxy group are the same as those specified above in connection with the foregoing lower alkyl group.

Examples of the cycloalkyl groups suitably used herein and appearing in the definition of the substituent R¹ include those having 3 to 10 carbon atoms and preferably 3 to 8 carbon atoms. Specific examples thereof suitably used herein are those derived from cyclopentane, cyclohexane and cycloheptane. These cycloalkyl groups may have any substituent and examples of such substituents are those listed above in connection with the foregoing lower alkyl group and the alkyl groups likewise listed above. The alkyl groups may be linear or branched ones, preferably used herein are lower alkyl groups and specific examples thereof suitably used herein are the same as those specified above in connection with the foregoing lower alkyl group.

Examples of the aryl groups suitably used herein and appearing in the definition of the substituent R¹ include phenyl and biphenyl groups, with phenyl groups being particularly preferred. The aryl group may have any substituent and examples of such substituents are the same as those listed above in connection with the foregoing lower alkyl group as well as alkyl groups and cycloalkyl groups. The alkyl groups may be linear or branched ones, preferably used herein are lower alkyl groups and specific examples thereof suitably used herein are the same as those specified above in connection with the foregoing lower alkyl group. In addition, specific examples of cycloalkyl groups as such substituents of the aryl groups are the same as those listed above in connection with the cycloalkyl groups appearing in the definition of the foregoing substituent R¹. The number of such substituents on the aryl group may range from 1 to 5 and preferably 1 to 3.

Examples of the aromatic heterocyclic groups suitably used herein and appearing in the definition of the substituent R¹ include thiazolyl groups, pyridyl groups, pyrimidyl groups, pyrazinyl groups, furyl groups, thienyl groups and pyrrolyl groups. The aromatic heterocyclic group may have any substituent and the substituents listed above in connection with the foregoing aryl group may likewise be used for the aromatic heterocyclic group without any particular restriction. Moreover, the number of such substituents on the aryl group may range from 1 to 5 and preferably 1 to 3.

R² represents a hydrogen atom, a halogen atom, a cyano group, a lower alkyl group, an aryl group or an aromatic heterocyclic group. Examples of the halogen atoms are the same as those listed above in connection with the foregoing substituent R¹.

Examples of the lower alkyl groups, the aryl groups or the aromatic heterocyclic groups suitably used herein and appearing in the definition of the substituent R² are the same as those described above in connection with the foregoing substituent R¹.

R³ represents a lower alkyl group or an aryl group and examples of these groups are the same as those specified above.

R⁴ represents a lower alkyl group, an aryl group, -C(=O)-R⁶, or -CH(OH)-R⁶ (in these formulas, R⁶ represents a lower alkyl group or an aryl group). In this respect, examples of the lower alkyl groups or the aryl groups suitably used herein are the same as those listed above in connection with the foregoing substituent R¹.

R⁵ represents a hydrogen atom, a lower alkyl group or an aryl group. In this respect, examples of the lower alkyl groups or the aryl groups suitably used herein are the same as those listed above in connection with the foregoing substituent R¹.

The 2-pyridone derivative according to the present invention may directly be used without any post treatment or may be used in the form of a pharmaceutically acceptable salt thereof or further a pro-drug thereof.

The salt of the 2-pyridone derivative according to the present invention is not restricted to any particular one, but examples thereof preferably used herein are acid-addition salts with mineral acids such as hydrochlorides, sulfates, nitrates and phosphates; and acid-addition salts with organic acids such as acetates, benzoates, fumarates, maleates, tartrates, citrates, lactates, oxalates, methane-sulfonates, benzene-sulfonates, and p-toluene-sulfonates.

In this connection, examples of metals constituting the salts of the 2-pyridone derivative according to the present invention are preferably alkali and alkaline earth metals such as sodium, potassium, and calcium.

When using, as a pro-drug, the 2-pyridone derivative according to the present invention, whose functional group is protected, the protected compound is not restricted to any specific one inasmuch as it is pharmaceutically acceptable one, but examples thereof include the derivatives whose hydroxyl group is protected, for instance, those converted into ethers or esters such as those in their etherified or esterified forms, and diol type protected derivatives; the derivatives, whose carbonyl group is protected, such as acetal type protected derivatives and ketal type protected derivatives; the derivatives, whose carboxyl group is protected, such as protected derivatives in the form of their esters, amides and hydrazides; the derivatives, whose thiol group is protected, such as protected derivatives in the form of their thioesters and thioethers; and the derivatives, whose amino group is protected, such as protected derivatives in the form of their carbamates, amides, allylamines, benzylamines and nitro-benzylamines.

Then the method for preparing the 2-pyridone derivative according to the present invention will be described in detail below.

The 2-pyridone derivative according to the present invention can be prepared according to, for instance, the methods A to C detailed below. In this respect, however, the preparation method C is herein given only for reference.

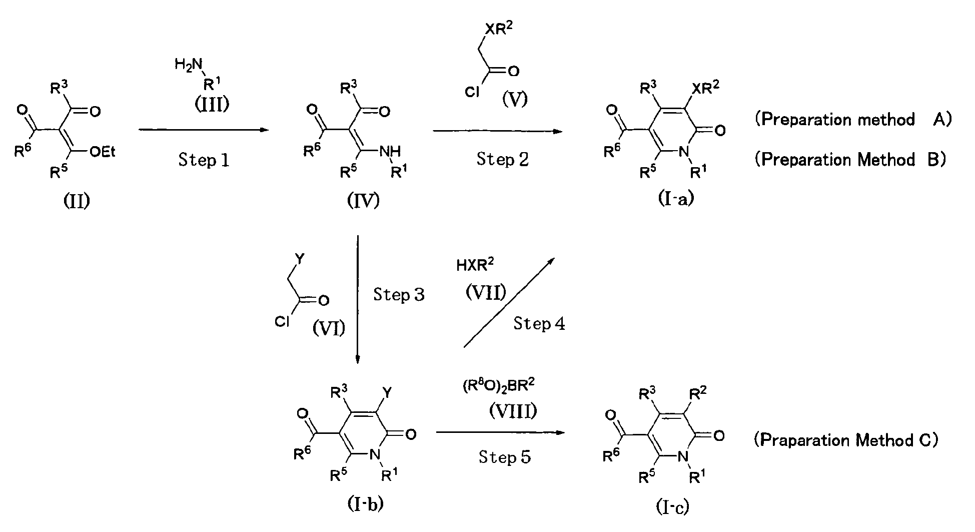

In the formulas, X represents S; and R¹, R², R³, R⁵, and R⁶ are the same as those specified above in connection with the compound represented by the foregoing general formula (1). In addition, Et represents an ethyl group; Y represents a halogen atom; and R⁸ represents a hydrogen atom, an alkyl group or an aryl group).

### (Preparation Method A)

### (Step 1):

A compound (IV) can be prepared by reacting a compound (III) with a compound (II) which can be prepared by any known method (for instance, those disclosed in, for instance, U.S. Patent No. 2,824,121; Mem. Fac. Eng., Osaka City Univ. 21, 175-181, 1980; Z. Naturforsch, B Anorg., Chem. Org. Chem., 34, 283-289, 1979; Chem. Ber., 114: 3471-3484, 1981) or according to any one of the methods disclosed in the foregoing articles, in the presence or absence of an inert solvent. The compound (III) used herein may be a commercially available one (such as those available from Aldrich Company) or can be prepared by any known method (for instance, those described in, for instance, S. R. Sandler and W Karo, "Organic Functional Group Preparations", vol.1, Academic (1983), p. 377) or according to any one of the methods disclosed in the foregoing article.

In this connection, examples of such inert solvents optionally used in the foregoing preparation methods suitably include alcohols such as methanol and ethanol; ethers such as tetrahydrofuran, diethyl ether, and dioxane; hydrocarbons such as benzene, toluene, and xylene; halogenated hydrocarbons such as chloroform, and methylene chloride; ketones such as acetone; polar organic solvents such as acetonitrile, N,N-dimethyl-formamide, and dimethyl-sulfoxide; or mixed solvents such as those comprising water and the foregoing organic solvents.

The reaction temperature may vary mainly depending on the kinds of starting compounds or solvents selected, but it in general ranges from 0 to 200°C and the reaction is suitably be carried out at a temperature ranging from room temperature to 120°C.

The reaction time may likewise vary mainly depending on the reaction temperature selected and the kinds of starting compounds or solvents selected, but it in general ranges from 5 minutes to 24 hours and suitably 15 minutes to one hour.

### (Step 2)

An intended compound (I-a) can be prepared by the reaction of a compound (IV) with a compound (V) in an inert solvent in the presence of a base. The compound (V) used herein may be a commercially available one (such as those available from Aldrich Company) or can be prepared by any known method (for instance, those described in, for instance, Collect. Czech. Chem. Commun., 1987, 52(7): 1811-1833) or according to any one of the methods disclosed in the foregoing article.

Examples of such inert solvents used in this step 2 include ethers such as tetrahydrofuran, diethyl ether, and dioxane; polar organic solvents such as N,N-dimethylformamide; hydrocarbons such as benzene, toluene, and xylene; or mixture thereof.

Examples of the bases suitably usable herein include organic bases such as triethylamine, diisopropyl-ethylamine, and pyridine; inorganic bases such as potassium carbonate, sodium hydrogen carbonate, sodium hydroxide, and sodium hydride; and metal alkoxides such as sodium methoxide, and potassium t-butoxide.

The reaction temperature may vary mainly depending on the kinds of starting compounds or solvents selected, but it in general ranges from 0 to 200°C and the reaction is suitably carried out at a temperature ranging from room temperature to 120°C.

The reaction time may likewise vary mainly depending on the reaction temperature selected and the kinds of starting compounds or solvents selected, but it in general ranges from 1 to 48 hours and suitably 12 to 24 hours.

### (Preparation Method B)

### (Step 3)

An intended compound (I-b) can be prepared by the reaction of the compound (IV) obtained in the step 1 of Preparation Method A with a compound (VI) in an inert solvent in the presence of a base, as in the foregoing Step 2. The compound (VI) used in this step may be a commercially available one (such as those available from Aldrich Company) or can be prepared by any known method (for instance, those described in, for instance, Org. Synth. IV 715, 1963) or according to any one of the methods disclosed in the foregoing article.

### (Step 4)

An intended compound (I-a) can be prepared by the reaction of the compound (I-b) with a compound (VII) in an inert solvent in the presence or absence of a base, while using a catalyst, if necessary.

In this connection, examples of such inert solvents used in this step suitably include alcohols such as methanol and ethanol; ethers such as tetrahydrofuran, diethyl ether, and dioxane; hydrocarbons such as benzene, toluene, and xylene; halogenated hydrocarbons such as chloroform, and methylene chloride; polar organic solvents such as N,N-dimethylformamide; or mixed solvents such as those comprising water and the foregoing organic solvents.

Examples of the bases suitably usable herein include organic bases such as triethylamine, diisopropyl-ethylamine, and pyridine; inorganic bases such as potassium carbonate, sodium hydrogen carbonate, sodium hydroxide, and sodium hydride; and metal alkoxides such as sodium methoxide, and potassium t-butoxide.

Examples of the catalysts suitably usable herein include powdery copper, copper salts such as monovalent copper halide, monovalent copper oxide, and divalent copper oxide, palladium chloride, palladium acetate and 0-valent palladium catalyst.

The reaction temperature may vary mainly depending on the kinds of starting compounds or solvents selected, but it in general ranges from 0 to 250°C and the reaction is suitably carried out at a temperature ranging from 80 to 160°C.

The reaction time may likewise vary mainly depending on the reaction temperature selected and the kinds of starting compounds or solvents selected, but it in general ranges from 1 to 48 hours and suitably 3 to 24 hours.

### (Preparation Method C) (Given for Reference)

### (Step 5)

The desired compound (I-c) can be prepared by the reaction of the compound (I-b) with a boronic acid (VIII) by any known method (such as those disclosed in, for instance, Chem. Rev. 95, 2457-2483, 1995) or according to any one of the methods disclosed in the foregoing article. The boronic acid (VIII) used herein may be a commercially available one (such as those available from Aldrich Company) or can be prepared by any known method (for instance, those described in, for instance, J. Am. Chem. Soc., 87, 1526, 1965; Tetrahedron Lett., 49, 7113, 7115, 1990) or according to any one of the methods disclosed in the foregoing articles.

### (Preparation Method D)

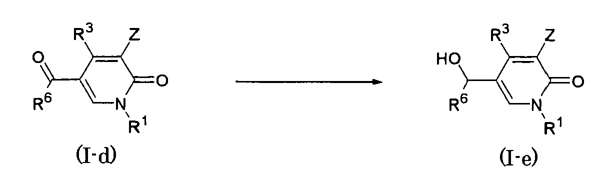

In the formulae, Z represents -SR² and R¹, R², R³, and R⁶ are the same as those specified above in connection with the compound represented by the foregoing formula (1).

The desired compound (I-e) can be prepared by the reaction of a compound (I-d) (corresponding to a compound represented by the general formula (I-a) wherein R⁵ represents a hydrogen atom) prepared according to the foregoing preparation method A or B, in a solvent, for instance, an alcohol such as methanol or ethanol; an ether such as tetrahydrofuran, diethyl ether or dioxane; water; or mixture thereof, in the presence of a reducing agent, for instance, metal hydride such as lithium aluminum hydride, sodium boron hydride, or a boron hydride compound such as boranes, generally at a temperature ranging from 0°C to room temperature for a time ranging from 5 minutes to 24 hours.

### (Preparation Method E)

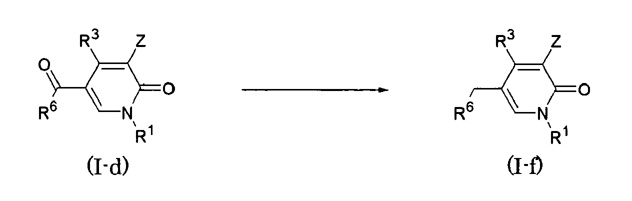

In the formulas, Z represents -SR² and R¹, R², R³, and R⁶ are the same as those specified above in connection with the compound represented by the foregoing general formula (1).

The desired compound (I-f) can be prepared by the reaction of a compound (I-d) (corresponding to a compound represented by the formula (I-a) wherein R⁵ represents a hydrogen atom) prepared according to the foregoing preparation method A or B, in a solvent, for instance, an alcohol such as methanol or ethanol; an ether such as tetrahydrofuran, diethyl ether or dioxane; water; or mixture thereof, optionally in the presence of an acid such as hydrochloric acid, acetic acid or p-toluene-sulfonic acid, using a metal such as tin, zinc, aluminum, copper, nickel or palladium, or a metal salt or an alloy thereof, generally at a temperature ranging from 0°C to 120°C for a time ranging from 1 to 48 hours.

### (Preparation Method F)

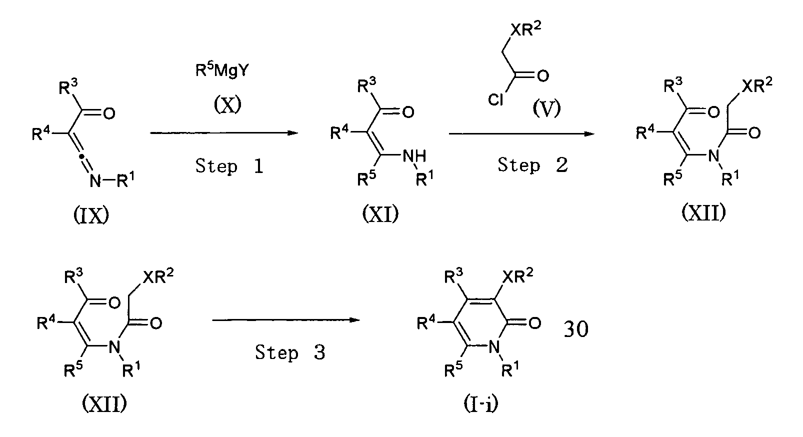

In the formulas, X represents S and R¹, R², R³, R⁴ and R⁵ are the same as those specified above in connection with the compound represented by the foregoing general formula (1), and Y represents a halogen atom.

### (Step 1)

A desired compound (XI) can be prepared by the reaction of a compound (IX) with a compound (X) by any known method (such as those disclosed in, for instance, Tetrahedron Lett. 52, 5167, 1973) or according to any one of the methods disclosed in the foregoing article. The compound (IX) used herein can be prepared by any known method (for instance, those described in, for instance, J. Am. Chem. Soc., 68, 988, 1946) or according to any one of the methods disclosed in the foregoing article. The compound (X) used herein may be a commercially available one (such as those available from Aldrich Company) or can be prepared according to any known method.

### (Step 2)

An intended compound (XII) can be prepared by the reaction of a compound (IX) with a compound (V) by the method similar to that described in Step 2 of Preparation Example A, in an inert solvent in the presence of a base. Alternatively, the compound (XII) can be prepared by the reaction of the compound (IX) prepared in Step 1, without any post-treatment (isolation), with compound (V) in the same reaction system used in Step 1.

### (Step 3)

An intended compound (I-i) can be prepared by the reaction of a compound (XII) in a solvent, for instance, an alcohol such as methanol or ethanol; an ether such as tetrahydrofuran, diethyl ether, or dioxane; a hydrocarbon such as benzene, toluene, or xylene; a halogenated hydrocarbon such as chloroform, or methylene chloride; a ketone such as acetone; a polar organic solvent such as acetonitrile, N,N-dimethyl-formamide or dimethyl-sulfoxide; in the presence of a base, for instance, an organic base such as triethylamine, diisopropyl-ethylamine, or pyridine; an inorganic base such as potassium carbonate, sodium hydrogen carbonate, sodium hydroxide, or sodium hydride; or a metal alkoxide such as sodium methoxide, or potassium t-butoxide, generally at a temperature ranging from 0 to 120°C for a time ranging from 1 to 48 hours.

The 2-pyridone derivative according to the present invention is useful for preventing and/or treating disease conditions associated with leukocytic infiltration.

In addition, the 2-pyridone derivative according to the present invention is also useful for the preparation of a pharmaceutical composition showing a cell adhesion- inhibitory effect.

Moreover, the 2-pyridone derivative according to the present invention is also useful for preventing and/or treating dermatitis, allergies, asthmas, metastasis of cancer, rejections against transplantation, chronic rheumatoid arthritis, arteriosclerosis, acute respiratory distress syndrome (ADRS), and ischemia reperfusion.

In addition, the 2-pyridone derivative according to the present invention is also useful for preventing and/or treating ulcerative colitis, Crohn disease, multiple sclerosis, systemic lupus erythemathosus (SLE), psoriasis, and osteoporosis.

As the disease conditions associated with leukocytic infiltration, there can specifically be listed, for instance, inflammation, dermatitis, allergies, asthmas, metastasis of cancer, rejections against transplantation, chronic rheumatoid arthritis, arteriosclerosis, acute respiratory distress syndrome (ADRS), ischemia reperfusion, ulcerative colitis, Crohn disease, multiple sclerosis, systemic lupus erythemathosus (SLE), psoriasis, osteoporosis, diabetes, and autoimmune diseases.

The 2-pyridone derivative according to the present invention can be administered to an animal or a man as it is or in combination with a currently used pharmaceutically acceptable carrier. For instance, the effective daily dose thereof suitably ranges from 0.01 to 1000 mg/kg body weight/day and preferably 0.1 to 500 mg/kg body weight/day and the daily dose may be administered at a time or over several times in portions.

Alternatively, the 2-pyridone derivative according to the present invention is suitably incorporated into the pharmaceutical composition, for instance, in an amount ranging from 0.01 to 100% by mass and preferably 0.01 to 50% by mass on the basis of the total mass of the composition.

The pharmaceutical composition of the present invention may be formed into a variety of dosage forms. For instance, the pharmaceutical composition can be formed into pharmaceutical preparations in a variety of dosage forms including, for instance, those administered through the oral route such as tablets, capsules, creams, fine granules and powders; and those administered through the parenteral route such as injections and suppositories.

In the present invention, the pharmaceutical composition may further comprise a variety of additives depending on the dosage forms thereof, for instance, excipients such as sucrose, lactose and crystalline cellulose; binders such as hydroxypropyl cellulose, polyvinyl pyrrolidone, and sodium carboxymethyl cellulose; disintegrators such as hydroxypropyl cellulose having a low degree of substitution, carboxymethyl cellulose and croscarmellose sodium; surfactants such as sodium lauryl sulfate, Pluronic, and polysorbate; lubricants such as magnesium stearate, talc, and sucrose esters of fatty acids; fluidity-improving agents such as silicon dioxide and anhydrous potassium hydrogen phosphate; thickening agents such as sodium alginate, locust bean gum and sodium polyacrylate; diluents such as physiological saline, and glucose aqueous solutions; corrigents; humectants; colorants; anti-microbials; preservatives; and perfumes.

In addition, the pharmaceutical composition of the present invention may likewise be formed into each desired dosage form, if necessary, while it is arbitrarily combined with other medicines.

### Examples

The present invention will hereunder be described in more detail with reference to the following Examples and Test Examples, but the present invention is not restricted to these specific Examples and Test Examples at all. In the following Examples and Test Examples, the symbol: "m.p." is the abbreviation of melting point. Moreover, in the ¹H NMR spectroscopic measurements, various conditions of signals are represented by s (singlet state), d (doublet state), t (triplet state), q (quadruplet state), quint (quintuplet state), sext (sextuplet state), sept (septuplet state), m (multiplet state) and br (broad) or the like.

### Example 1: Preparation of 5-Acetyl-3-(4-Fluoro-Phenylthio)-4-Methyl-1-Phenyl-1H-Pyridin-2-One (Compound 1 as the compound No. 1 given in the following Table (those in the following description are shown in the same way also)) (corresponding to the compound (I-a)) (Preparation Method A):

### (a) Step 1: Preparation of 3-phenylaminomethylene-pentan-2,4-dione (corresponding to the compound (IV) in which R⁵ is a hydrogen atom) (Compound (a)):

To aniline (3.0 mL, 33.0mmol; corresponding to the compound (III)), there was added 3-ethoxymethylene-pentan-2,4-dione (6.69 g, 42.8mmol; corresponding to the compound (II)) with stirring at room temperature. Immediately after the completion of the addition, this reaction system underwent the generation of heat and the whole of the reaction system was completely solidified after about 10 minutes. After drying the reaction system under a reduced pressure, it was purified by the silica gel column chromatography (eluted with hexane: ethyl acetate = 3:1) to thus give the title compound (a) as white crystals (yield: 6.78 g; quantitative). Further, the product was recrystallized from hexane/ethyl acetate to thus obtain the title compound (a) as colorless needles.
m.p. 86.2-87.0°C; ¹H NMR (400MHz, CDCl₃) δ ; 2.39 (3H, s), 2.57 (3H, s), 7.19 (2H, d, J=7.6Hz), 7.23 (1H, t, J=7.6Hz), 7.42 (2H, t, J=7.6Hz), 8.25 (1H, d, J=13.0Hz), 12.76 (1H, brd, J=13.0Hz)

### (b) Step 2: Preparation of 5-acetyl-3-(4-fluoro-phenylthio)-4-methyl-1-phenyl-1H-pyridin-2-one (Compound 1):

To a solution of 3-phenylaminomethylene-pentan-2,4-dione (Compound (a); 3.82 g, 18.8mmol) in tetrahydrofuran (100ml), there was added a 60% sodium hydride (2.0 g, 50.0mmol) followed by stirring the resulting mixture for 40 minutes. Thereafter, to the mixture, there was added (4-fluoro-phenylthio) acetyl chloride (corresponding to the compound (V); 3.80 g, 24.4mmol) and the reaction between them was then continued for 16 hours. To the reaction system, there was added water, followed by the stirring of the mixture for 10 minutes and then the addition of a saturated aqueous ammonium chloride solution to the mixture. The reaction mixture was extracted twice with ethyl acetate, followed by the washing of the combined organic phases with a saturated aqueous sodium chloride solution, drying the combined organic phase over anhydrous sodium sulfate and the subsequent removal of the solvent. The resulting residue was purified by the silica gel column chromatography (eluted with hexane: ethyl acetate = 3:1) to thus give the title compound 1 as a foam (3.49 g, 57.6%). Further, the product was recrystallized from hexane/chloroform to thus obtain the title compound 1 as colorless rectangular crystal.
m.p. 124.8-126.6°C; ¹H NMR (400MHz, CDCl₃) δ ; 2.47 (3H, s), 2.84 (3H, s), 6.89-6.96 (2H, m), 7.29-7.37 (4H, m), 7.43-7.53 (3H, m), 8.03 (1H, s); ¹³C NMR (100MHz, CDCl₃) δ ; 21.0, 28.5, 115.9, 116.1, 119.6, 126.4, 129.6, 131.4, 131.5, 140.3, 142.8, 155.6, 159.8, 160.6, 163.0, 195.1.

### Example 2: (5-Acetyl-3-(4-Fluoro-phenylthio)-1-(4-Methoxybenzyl)-4-Methyl-1H-Pyridin-2-one) (Compound 31; corresponding to the compound (I-a)) (Preparation Example A)

### (a) Step 1: Preparation of 3-[(4-methoxy-benzylamino)-methylene]-pentan-2,4-dione (Compound (b); corresponding to the compound (IV))

To 4-methoxy-benzylamine (corresponding to the compound (III); 1.40 g, 10.7 mmol), there was added 3-ethoxymethylene-pentan-2,4-dione (corresponding to the compound (II); 2.0 g, 12.9 mmol) at room temperature with stirring. After the stirring of the mixture for 30 minutes, it was dried under a reduced pressure, followed by washing the resulting crystals separated from the mixture with hexane and ethyl acetate in this order, again drying the crystals to thus quantitatively obtain crystalline 3-[(4-methoxy-benzylamino)-methylene]-pentan-2,4-dione (Compound (b); 2.68 g). Further, the crystalline product was recrystallized from hexane/ethyl acetate to thus obtain the compound (b) as yellow orthorhombic crystals.
m.p. 91.2-92.2°C; ¹H NMR (400MHz, CDCl₃) δ ; 2.24 (3H, s), 2.48 (3H, s), 3.81 (3H, s), 4.47 (2H, d, J=5.0Hz), 6.88-6.93 (2H, m), 7.16-7.21 (2H, m), 7.79 (1H, d, J=12.6Hz), 11.26 (1H, br).

### (b) Step 2: Preparation of 5-acetyl-3-(4-fluorophenylthio)-1-(4-methoxybenzyl)-4-methyl-1H-pyridin-2-one (Compound 31)

The foregoing compound (b) (648mg, 2.62mmol) was treated by the same procedures used above in Example 1(b) to thus give the title compound 31 as a paste-like product (580mg, 55.8%).
¹H NMR (400MHz, CDCl₃) δ; 2.37 (3H, s), 2.73 (3H, s), 3.81 (3H, s), 5.09 (2H, s), 6.86-6.96 (4H, m), 7.19-7.27 (4H, m), 7.91 (1H, s).

### Example 3: (Elimination Reaction at Position 1): Preparation of 5-acetyl-3-(4-fluoro-phenylthio)-4-methyl-1H-pyridin-2-one (Compound 42)

The foregoing compound (Compound 31; 458mg, 1.15mmol) was dissolved in trifluoroacetic acid (57.5mL) and the resulting solution was refluxed with stirring for 16 hours. The reaction mixture was concentrated and the residue was purified by the silica gel column chromatography (gradient elution using chloroform: ethyl acetate (10:1) and chloroform: methanol (10:1)) to thus give the title compound 42 (241mg, 75.5%) as yellow crystals.
m.p.236-238°C (chloroform/methanol) ¹H NMR (400MHz, CDCl₃) δ ; 2.47 (3H, s), 2.76 (3H, s), 6.93-6.97 (2H, m), 7.17-7.21 (2H, m), 8.07 (1H, s), 12.55 (1H, br).

### Example 4: 5-Acetyl-3-(4-fluoro-phenylthio)-4,6-dimethyl-1-phenyl-1H-pyridin-2-one (Compound 62; corresponding to the compound (I-a)) (Preparation Method A)

### (a) Step 1: Preparation of 3-(phenylamino-ethylidene)-pentan-2,4-dione (Compound (c))

3-(Ethoxy-ethylidene)-pentan-2,4-dione (Russian Journal of General Chemistry, Vol. 64, No. 1, Part 2, 1994, p. 123; corresponding to the compound (II); 2.49mg, 14.6mmol) was dissolved in toluene (75mL), an aniline (corresponding to the compound (III); 1.64mg, 17.5mmol) was then added to the resulting solution and the mixture was refluxed with stirring over 16 hours. After allowing the mixture to cool, the reaction mixture was concentrated and then purified by the silica gel chromatography (eluted with hexane: ethyl acetate = 3:1) to thus give the title compound (c) (1.15g, 36.3%) as a pale yellow syrup.
¹H NMR (400MHz, CDCl₃) δ ; 2.05 (3H, s), 2.25 (3H, brs), 2.46 (3H, brs), 7.10-7.14 (2H, m), 7.17-7.21 (1H, m), 7.36-7.41 (2H, m), 13.73 (1H, brs).

### (b) Step 2: Preparation of 5-acetyl-3-(4-fluoro-phenylthio)-4,6-dimethyl-1-phenyl-1H-pyridin-2-one (Compound 62)

The foregoing compound (c) (450mg, 2.07mmol) was treated by the same procedures used in Example 1(b) to thus give the title compound 62 (499mg, 65.6%) as a paste-like product.
¹H NMR (400MHz, CDCl₃) δ ; 1.91 (3H, s), 2.47 (3H, s), 2.52 (3H, s), 6.87-6.95 (2H, m), 7.11-7.16 (2H, m), 7.25-7.34 (2H, m), 7.40-7.51 (3H, m).

The compounds (the compounds of the present invention and reference compounds) prepared by the same procedures used in these Examples are summarized in the following Table 1. In this respect, various symbols appearing in the following Table 1 are as follows: Ph: phenyl group; Me: methyl group; Et: ethyl group; Bn: benzyl group; iBu: isobutyl group; nBu: normal butyl group.

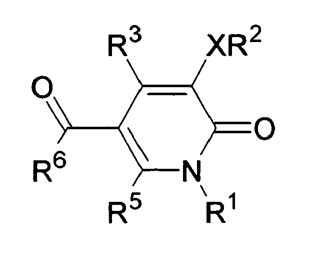

**Table 1**

| Compound No. | R¹ | X | R² | R³ | R⁶ | R⁵ |
|---|---|---|---|---|---|---|
| 1 | Ph | S | 4-F-Ph | Me | Me | H |
| 2 | 3-pyridyl | S | 4-F-Ph | Me | Me | H |
| 3 | 3-pyridyl N-oxide | S | 4-F-Ph | Me | Me | H |
| 4 | 2-Cl-Ph | S | 4-F-Ph | Me | Me | H |
| 5 | 2-Br-Ph | S | 4-F-Ph | Me | Me | H |
| 6 | 2,4-di-Cl-Ph | S | 4-F-Ph | Me | Me | H |
| 7 | 4-Br-2-Cl-Ph | S | 4-F-Ph | Me | Me | H |
| 8 | 2,6-di-Cl-Ph | S | 4-F-Ph | Me | Me | H |
| 9 | 2-Cl-4-NO₂-Ph | S | 4-F-Ph | Me | Me | H |
| 10 | 2-Cl-4-NH₂-Ph | S | 4-F-Ph | Me | Me | H |
| 11 | 2-Cl-6-CO₂H-Ph | S | 4-F-Ph | Me | Me | H |
| 12 | 2-Cl-3-CO₂H-Ph | S | 4-F-Ph | Me | Me | H |
| 13 | 3-Cl-4-CO₂Me-Ph | S | 4-F-Ph | Me | Me | H |
| 14 | 3-Cl-4-CO₂H-Ph | S | 4-F-Ph | Me | Me | H |
| 15 | 4-Br-Ph | S | 4-F-Ph | Me | Me | H |
| 16 | 4-Br-2-Me-Ph | S | 4-F-Ph | Me | Me | H |
| 17 | 2,6-di-Me-Ph | S | 4-F-Ph | Me | Me | H |
| 18 | Ph | S | 4-F-Ph | Et | Et | H |
| 19 | 2-Cl-Ph | S | 4-F-Ph | Et | Et | H |
| 20 (Reference) | Ph | CH₂ | Ph | Me | Me | H |
| 21 | cyclohexyl | S | 4-F-Ph | Me | Me | H |
| 22 | 5-Ph-2-thiazolyl | S | 4-F-Ph | Me | Me | H |
| 23 | 2,2-diethoxyethyl | S | 4-F-Ph | Me | Me | H |
| 24 | Bn | S | 4-F-Ph | Me | Me | H |
| 25 | 2-Cl-Bn | S | 4-F-Ph | Me | Me | H |
| 26 | 2-OPh-Ph | S | 4-F-Ph | Me | Me | H |
| 27 | 2-OMe-Ph | S | 4-F-Ph | Me | Me | H |
| 28 | 3-OMe-Ph | S | 4-F-Ph | Me | Me | H |
| 29 | 4-OMe-Ph | S | 4-F-Ph | Me | Me | H |
| 30 (Reference) | Ph | (XR² | = R² = H) | Me | Me | H |
| 31 | 4-OMe-Bn | S | 4-F-Ph | Me | Me | H |
| 32 | 2-OH-Ph | S | 4-F-Ph | Me | Me | H |
| 33 | 3-OBn-2-pyridyl | S | 4-F-Ph | Me | Me | H |
| 34 | cyclopropyl | S | 4-F-Ph | Me | Me | H |
| 35 (Reference) | Ph | O | Me | Me | Me | H |
| 36 | 2-pyrimidinyl | S | 4-F-Ph | Me | Me | H |
| 37 | 2-pyrazinyl | S | 4-F-Ph | Me | Me | H |
| 38 | 3-pyridyl N-oxide | S | Ph | Me | Me | H |
| 39 | 2,4-dimethoxy-Ph | S | 4-F-Ph | Me | Me | H |
| 40 | 3,4- dimethoxy-Ph | S | 4-F-Ph | Me | Me | H |
| 41 | 3,5- dimethoxy-Ph | S | 4-F-Ph | Me | Me | H |
| 42 | H | S | 4-F-Ph | Me | Me | H |
| 43 | Ph-3-propyl | S | 4-F-Ph | Me | Me | H |
| 44 | -CH₂-COOEt | S | 4-F-Ph | Me | Me | H |
| 45 | -CH₂-COOH | S | 4-F-Ph | Me | Me | H |
| 46 | -CH₂-CONH-Ph | S | 4-F-Ph | Me | Me | H |
| 47 | Ph | S | 4-F-Ph | i-Bu | Me | H |
| 48 | Ph | S | 4-F-Ph | Me | i-Bu | H |
| 49 | Ph | S | 4-F-Ph | Ph | Me | H |
| 50 | Ph | S | 4-F-Ph | Me | Ph | H |
| 51 | Ph | S | 4-F-Ph | n-Bu | Me | H |
| 52 | Ph | S | 4-F-Ph | Me | n-Bu | H |
| 53 | 4-NO₂-Ph | S | 4-F-Ph | Me | Me | H |
| 54 | 4-NH₂-Ph | S | 4-F-Ph | Me | Me | H |
| 55 (Reference) | Ph | O | 4-F-Ph | Me | Me | H |
| 56 | Ph | S | Ph | Me | Me | H |
| 57 | 3,4-dimethyl-Ph | S | 4-F-Ph | Me | Me | H |
| 58 | 2-pyridyl | S | 4-F-Ph | Me | Me | H |
| 59 | cyclooctyl | S | 4-F-Ph | Me | Me | H |
| 60 | 2-pyridyl·HCl | S | 4-F-Ph | Me | Me | H |
| 61 | 4-pyridyl | S | 4-F-Ph | Me | Me | H |
| 62 | Ph | S | 4-F-Ph | Me | Me | Me |
| 63 | 4-pyridyl·HCl | S | 4-F-Ph | Me | Me | H |
| 64 | Ph | S | 4-F-Ph | Ph | Ph | Ph |
| 65 | Ph | S | 4-F-Ph | Et | Et | Et |
| 66 | 3-OMe-Ph | S | 4-F-Ph | Me | Me | Me |
| 67 | 3-OMe-Ph | S | 4-OMe-Ph | Me | Me | Me |
| 68 | 3-OH-Ph | S | 4-F-Ph | Me | Me | Me |
| 69 | Ph | S | 4-OMe-Ph | Me | Me | Me |

The results of ¹H NMR spectroscopic measurements of the foregoing compounds are listed in the following Table 2.

**Table 2**

| Compound No. | ¹H NMR { CDCl₃/TMS, δ (ppm) } |
|---|---|
| 2 | 2.49 (3H, s), 2.84 (3H, s), 6.92-6.97 (2H, m), 7.28-7.33 (2H, m), 7.45 (1H, ddd, J=1.0, 4.7, 9.0Hz), 7.80 (1H, ddd, J=1.8, 2.5, 9.0Hz), 7.99 (1H, s), 8. 63 (1H, d, J=2.5Hz), 8.70 (1H, dd, J=1.8, 4.7Hz). |
| 3 | 2.50 (3H, s), 2.83 (3H, s), 6.93-6.98 (2H, m), 7.28-7.40 (4H, m), 7.90 (1H, s,), 8.23 -8.29 (2H, m). |
| 4 | 2.47 (3H, s), 2.84 (3H, s), 6.89-6.96 (2H, m), 7.25-7.31 (2H, m), 7.34-7.46 (3H, m), 7.53-7.57 (1H, m), 7.88 (1H, s). |
| 5 | 2.47 (3H, s), 2.85 (3H, s), 6.89-6.96 (2H, m), 7.21-7.38 (4H, m), 7.42-7.48 (1H, m), 7.69-7.74 (1H, m), 7.86 (1H, s). |
| 6 | 2.46 (3H, s), 2.83 (3H, s), 6.90-6.96 (2H, m), 7.26-7.31 (3H, m), 7.39 (1H, dd, J=2.5, 9.3Hz), 7.57 (1H, d, J=2.5Hz), 7.82 (1H, s). |
| 7 | 2.50 (3H, s), 2.84 (3H, s), 6.90-6.93 (2H, m), 7.23 (1H, d, J=8.6Hz), 7.23-7 .31 (2H, m), 7.54 (1H, dd, J=2.5, 8.6Hz), 7.72 (1H, d, J=2.5Hz), 7.84 (1H, s). |
| 8 | 2.45 (3H, s), 2.83 (3H, s), 6.87-6.93 (2H, m), 7.21-7.26 (2H, m), 7.35 (1H, dd, J=7.5, 9.3Hz), 7.45 (1H, d, J=7.5Hz), 7.46 (1H, d, J=9.3Hz), 7.74 (1H, s). |
| 9 | 2.46 (3H, s), 2.82 (3H, s), 6.89-6.95 (2H, m), 7.26-7.30 (2H, m), 7.57 (1H, d, J=9.0Hz), 7.78 (1H, s), 8.25 (1H, dd, J=2.9, 9.0Hz), 8.41 (1H, d, J=2.9 Hz). |
| 10 | 2.43 (3H, s), 2.80 (3H, s), 3.92 (2H, br), 6.57 (1H, dd, J=2.9, 8.6Hz), 6.74 (1H, d, J=2.9Hz), 6.86-6.93 (2H, m), 7.05 (1H, d, J=8.6Hz), 7.22-7.27 (2H, m), 7.87 (1H, s). |
| 11 | 2.41 (3H, s), 2.77 (3H, s), 4.84 (1H, br), 6.81-6.89 (2H, m), 7.11 -7.17 (2H , m), 7.39-7.48 (1H, m), 7.65-7.72 (1H, m), 7.87 (1H, s,), 7.87 -7.95 (1H, m). |
| 12 | 2.48 (3H, s), 2.85 (3H, s), 2.10-3.10 (1H, br), 6.90-6.97 (2H, m), 7.26-7.32 (2H, m), 7.66 (1H, d, J=8.6Hz), 7.87 (1H, s), 8.07 (1H, d, J=2.5Hz), 8.11 ( 1H, dd, J=2.5, 8.6Hz). |
| 13 | 2.49 (3H, s), 2.83 (3H, s), 3.96 (3H, s), 6.91-6.97 (2H, m), 7.28-7.33 (2H, m), 7.36 (1H, dd, J=2.2, 8.3Hz), 7.51 (1H, d, J=2.2Hz), 7.95 (1H, s), 7.97 ( 1H, d, J=8.3Hz). |
| 14 | 2.50 (3H, s), 2.83 (3H, s), 3.96 (3H, s), 6.91-6.98 (2H, m), 7.30-7.35 (2H, m), 7.39 (1H, dd, J=2.5, 8.3Hz), 7.55 (1H, d, J=2.5Hz), 7.96 (1H, s), 8.10 ( 1H, d, J=8.3Hz). |
| 15 | 2.48 (3H, s), 2.83 (3H, s), 6.90-6.96 (2H, m), 7.23-7.27 (2H, m), 7.28-7.34 (2H, m), 7.60-7.65 (2H, m), 7.98 (1H, s). |
| 16 | 2.03 (3H, s), 2.45 (3H, s), 2.84 (3H, s), 6.89-6.96 (2H, m), 7.05 (1H, d, J= 9.0Hz), 7.27-7.34 (2H, m), 7.43-7.50 (2H, m), 7.85 (1H, s). |
| 17 | 2.00 (6H, s), 2.42 (3H, s), 2.84 (3H, s), 6.86-6.92 (2H, m), 7.10-7.15 (2H, brd), 7.20-7.29 (3H, m), 7.77 (1H, s). |
| 18 | 1.19 (3H, t, J=6.5Hz), 1.24 (3H, t, J=6.5Hz), 2.79 (2H, q, J=6.5Hz), 3.34 (2 H, q, J=6.5Hz), 6.89-6.95 (2H, m), 7.26-7.35 (4H, m), 7.41-7.52 (3H, m), 8. 00 (1H, s). |
| 19 | 1.19 (3H, t, J=6.5Hz), 1.26 (3H, t, J=6.5Hz), 2.79 (2H, q, J=6.5Hz), 3.33 (2 H, q, J=6.5Hz), 6.88-6.95 (2H, m), 7.23-7.30 (2H, m), 7.31-7.43 (3H, m), 7. 51-7.55 (1H, m), 7.85 (1H, s). |
| 20 | 2.43 (3H, s), 2.52 (3H, s), 4.07 (2H, s), 7.13-7.30 (5H, m), 7.37-7.55 (5H, m), 7.93 (1H, s). |
| 21 | 1.16-1.29 (1H, m), 1.40-1.53 (4H, m), 1.78-1.81 (1H, m), 1.87-2.00 (4H, m), 2.51 (3H, s), 2.76 (3H, s), 4.82 (1H, tt, J=3.2, 7.9Hz), 6.90-6.96 (2H, m), 7.20-7.26 (2H, m), 7.99 (1H, s). |
| 22 | 2.69 (3H, s), 2.83 (3H, s), 6.93-6.99 (2H, m), 7.26-7.30 (2H, m), 7.36-7.41 (1H, m), 7.43-7.50 (3H, m), 7.90-7.95 (2H, m), 9.65 (1H, s). |
| 23 | 1.15 (6H, t, J=6.8Hz), 2.47 (3H, s), 2.77 (3H, s), 3.47 (1H, q, J=6.8Hz), 3. 49 (1H, q, J=6.8Hz), 3.53 (1H, q, J=6.8Hz), 3.55 (1H, q, J=6.8Hz), 4.05 (2 H, d, J=5.4Hz), 4.68 (1H, t, J=5.4Hz), 6.90-6.96 (2H, m), 7.13-7.23 (2H, m) , 8.08 (1H, s). |
| 24 | 2.38 (3H, s), 2.74 (3H, s), 5.17 (2H, s), 6.90-6.97 (2H, m), 7.19-7.40 (7H, m), 7.92 (1H, s). |
| 25 | 2.41 (3H, s), 2.75 (3H, s), 5.29 (2H, s), 6.89-6.95 (2H, m), 7.18-7.33 (4H, m), 7.39-7.45 (2H, m), 8.15 (1H, s). |
| 26 | 2.41 (3H, s), 2.78 (3H, s), 6.70-6.77 (2H, m), 6.88-6.93 (2H, m), 6.98-7.02 (1H, m), 7.09-7.30 (6H, m), 7.36-7.40 (2H, m), 8.01 (1H, s). |
| 27 | 2.44 (3H, s), 2.83 (3H, s), 3.77 (3H, s), 6.88-6.95 (2H, m), 7.02-7.07 (2H, m), 7.23-7.30 (3H, m), 7.42 (1H, dt, J=1.4, 8.0Hz), 7.94 (1H, s). |
| 28 | 2.47 (3H, s), 2.83 (3H, s), 3.82 (3H, s), 6.88-7.00 (5H, m), 7.27-7.34 (2H, m), 7.39 (1H, t, J=7.9Hz), 8.02 (1H, s). |
| 29 | 2.47 (3H, s), 2.83 (3H, s), 3.84 (3H, s), 6.90-7.00 (4H, m), 7.24-7.33 (4H, m), 8.02 (1H, s). |
| 30 | 2.43 (3H, s), 2.51 (3H, d, J=1.1Hz), 6.45 (1H, d, J=1.1Hz), 7.37-7.41 (2H, m), 7.46-7.56 (3H, m), 8.02 (1H, s). |
| 32 | 2.51 (3H, s), 2.84 (3H, s), 6.91-7.39 (8H, m), 8.06 (1H, s). |
| 33 | 2.45 (3H, s), 2.79 (3H, s), 5.10 (2H, s), 6.79-6.85 (2H, m), 7.22-7.41 (9H, m), 8.10 (1H, s), 8.17 (1H, dd, J=1.8, 4.7Hz). |
| 34 | 0.81-0.94 (2H, m), 1.10-1.24 (2H, m), 2.49 (3H, s), 2.75 (3H, s), 3.36 (1H, sept, J=3.2Hz), 6.90-6.95 (2H, m), 7.22-7.26 (2H, m), 7.97 (1H, s). |
| 35 | 2.44 (3H, s), 2.47 (3H, s), 3.90 (3H, s), 7.38-7.42 (2H, m), 7.45-7.56 (3H, m), 7.84 (1H, s). |
| 36 | 2.51 (3H, s), 2.82 (3H, s), 6.90-6.96 (2H, m), 7.29-7.34 (2H, m), 7.43 (1H, t, J=4.6Hz), 8.35 (1H, s), 8.89 (1H, d, J=4.6Hz). |
| 37 | 2.55 (3H, s), 2.84 (3H, s), 6.92-6.98 (2H, m), 7.26-7.32 (2H, m), 8.55 (1H, dd, J=1.1, 1.8Hz), 8.63 (1H, s), 8.63 (1H, d, J=1.8Hz), 9.33 (1H, d, J=1.1 Hz). |
| 38 | 2.50 (3H, s), 2.80 (3H, s), 7.15-7.28 (5H, m), 7.35-7.40 (2H, m), 7.92 (1H, s), 8.22-8.27 (1H, m), 8.28-8.30 (1H, m). |
| 39 | 2.44 (3H, s), 2.82 (3H, s), 3.74 (3H, m), 3.83 (3H, m), 6.88-6.94 (2H, m), 6.54 (1H, dd, J=1.8, 8.6Hz), 6.56 (1H, d, J=1.8Hz), 7.15 (1H, d, J=8.6Hz). 7.26-7.31 (2H, m), 7.93 (1H, s). |
| 40 | 2.47 (3H, s), 2.83 (3H, s), 3.87 (3H, s), 3.91 (3H, s), 6.84-6.96 (5H, m), 7. 28-7.34 (2H, m), 8.03 (1H, s). |
| 41 | 2.46 (3H, s), 2.82 (3H, s), 3.79 (6H, s), 6.47 (2H, d, J=2.2Hz), 6.52 (1H, t, J=2.2Hz), 6.89-6.95 (2H, m), 7.28-7.34 (2H, m), 8.01 (1H, s). |
| 43 | 2.13 (2H, quint, J=7.2Hz), 2.43 (3H, s), 2.67 (2H, t, J=7.2Hz), 2.74 (3H, s), 3.98 (3H, t, J=7.2Hz), 6.90-6.96 (2H, m), 7.14-7.31 (7H, m), 7.79 (1H, s). |
| 44 | 1.27 (3H, t, J=7.9Hz), 2.49 (3H, s), 2.77 (3H, s), 4.23 (2H, q, J=7.9Hz), 4. 69 (2H, s), 6.90-6.96 (2H, m), 7.18-7.23 (2H, m), 7.91 (1H, s). |
| 45 | (CD₃OD / CH₃OH = 3.30ppm) 2.50 (3H, s), 2.71 (3H, s), 4.78 (2H, s), 6.9 3-6.99 (2H, m), 7.13-7.18 (2H, m), 8.55 (1H, s). |
| 46 | 2.52 (3H, s), 2.78 (3H, s), 4.70 (2H, s), 6.88-6.93 (2H, m), 7.08-7.13 (1H, m), 7.18-7.34 (6H, m), 8.11 (1H, s), 8.72 (1H, brs). |
| 47 | 0.97 (6H, d, J=6.5Hz), 2.25 (1H, sept, J=6.5Hz), 2.60 (2H, d, J=6.5Hz), 2.7 9 (3H, s), 6.90-6.95 (2H, m), 7.29-7.37 (4H, m), 7.43-7.52 (3H, m), 7.94 (1 H, s). |
| 48 | 0.98 (6H, d, J=6.5Hz), 1.82 (1H, sept, J=6.5Hz), 2.48 (3H, s), 3.45 (2H, d, J=6.5Hz), 6.89-6.94 (2H, m), 7.25-7.35 (4H, m), 7.41-7.50 (3H, m), 8.01 (1 H, s). |
| 49 | 2.65 (3H, s), 6.92-6.98 (2H, m), 7.31-7.52 (9H, m), 7.58-7.64 (1H, m), 7.64 (1H, s), 7.80-7.85 (2H, m). |
| 50 | 1.78 (3H, s), 6.83-6.89 (2H, m), 7.16-7.20 (2H, m), 7.25-7.32 (10H, m), 8.0 2 (1H, s). |
| 51 | 0.93 (3H, t, J=6.5Hz), 1.36 (2H, sext, J=6.5Hz), 1.67 (2H, quint, J=6.5Hz), 2.74 (2H, t, J=6.5Hz), 2.79 (3H, s), 6.90-6.95 (2H, m), 7.29-7.36 (4H, m), 7.42-7.52 (3H, m), 7.97 (1H, s). |
| 52 | 0.98 (3H, brt, J=7.5Hz), 1.47-1.58 (4H, m), 2.48 (3H, m), 3.32-3.38 (2H, m ), 6.90-6.95 (2H, m), 7.27-7.35 (4H, m), 7.41-7.51 (3H, m), 8.04 (1H, s). |
| 53 | 2.50 (3H, s), 2.84 (3H, s), 6.92-6.98 (2H, m), 7.28-7.33 (2H, m), 7.57-7.61 (2H, m), 7.97 (1H, s), 8.34-8.39 (2H, m). |
| 54 | 2.46 (3H, s), 2.82 (3H, s), 3.85 (2H, br), 6.69-6.74 (2H, m), 6.88-6.95 (2H, m), 7.08-7.13 (2H, m), 7.26-7.33 (2H, m), 8.03 (1H, s). |
| 55 | 2.49 (3H, s), 2.50 (3H, s), 6.85-6.98 (4H, m), 7.36-7.54 (5H, m), 7.99 (1H, s). |
| 56 | 2.48 (3H, s), 2.81 (3H, s), 7.11-7.16 (1H, m), 7.19-7.27 (4H, m), 7.36-7.39 (2H, m), 7.42-7.52 (3H, m), 8.05 (1H, s). |
| 57 | 2.29 (6H, brs), 2.46 (3H, s), 2.83 (3H, s), 6.89-6.95 (2H, m), 7.06 (1H, dd, J=2.0, 8.6Hz), 7.12 (1H, d, J=2.0Hz), 7.24 (1H, d, J=8.6Hz), 7.28-7.33 (2H , m), 8.02 (1H, s). |
| 58 | 2.55 (3H, s), 2.83 (3H, s), 6.90-6.97 (2H, m), 7.21-7.30 (2H, m), 7.36 (1H, ddd, J=0.7, 4.7, 8.3Hz), 7.84 (1H, dt, J=2.2, 8.3Hz), 7.94 (1H, dd, J=0.7, 8 .3Hz), 8.57 (1H, dd, J=2.2, 4.7Hz), 8.70 (1H, s). |
| 59 | 1.50-1.93 (14H, m), 2.50 (3H, s), 2.74 (3H, s), 5.04-5.15 (1H, m), 6.90-6.97 (2H, m), 7.18-7.24 (2H, m), 7.98 (1H, s). |
| 61 | 2.50 (3H, s), 2.83 (3H, s), 6.91-6.98 (2H, m), 7.28-7.34 (2H, m), 7.35-7.38 (2H, m), 7.96 (1H, s), 8.76-8.80 (2H, m). |
| 64 | 6.85-7.34 (22H, m), 7.43-7.48 (2H, m). |
| 65 | 0.92 (3H, t, J=7.2Hz), 1.22 (3H, t, J=7.2Hz), 1.24 (3H, t, J=7.2Hz), 2.23 (2 H, q, J=7.2Hz), 2.76 (2H, q, J=7.2Hz), 2.82 (2H, q, J=7.2Hz), 6.87-6.95 (2 H, m), 7.14-7.19 (2H, m), 7.25-7.30 (2H, m), 7.39-7.50 (3H, m). |
| 66 | 1.95 (3H, s), 2.46 (3H, s), 2.52 (3H, s), 3.79 (3H, s), 6.64-6.67 (1H, m), 6. 69-6.73 (1H, m), 6.89-6.98 (3H, m), 7.27-7.33 (2H, m), 7.38 (1H, t, J=8.2H z), |
| 67 | 1.92 (3H, s), 2.47 (3H, s), 2.50 (3H, s), 3.76 (3H, s), 3.79 (3H, s), 6.64-6.6 6 (1H, m), 6.69-6.73 (1H, m), 6.75-6.80 (2H, m), 6.93-6.97 (1H, m), 7.31-7. 40 (3H, m). |
| 68 | 1.92 (3H, s), 2.49 (3H, s), 2.53 (3H, s), 6.19 (1H, brt, J=2.2Hz), 6.49 (1H, dd, J=2.2, 8.0Hz), 6.56 (1H, dd, J=2.2, 8.0Hz), 6.83-6.89 (2H, m), 7.20 (1H, t, J=8.0Hz), 7.25-7.31 (2H, m). |
| 69 | 1.89 (3H, s), 2.47 (3H, s), 2.50 (3H, s), 3.76 (3H, s), 6.75-6.80 (2H, m), 7. 10-7.15 (2H, m), 7.30-7.35 (2H, m), 7.39-7.50 (3H, m). |

### Example 5 (Reference):

### Preparation (Preparation Method B; Step 3) of 5-acetyl-3-bromo-4-methyl-1-phenyl-1H-pyridin-2-one (Compound 70; corresponding to the compound (I-b))

The same procedures used in Example 1(b) were repeated except for using the compound (a) prepared in Example 1(a) and bromo-acetyl chloride (corresponding to the compound (VI)) as raw materials to thus give the title compound 70 (reference compound) (41.4%) as white needles.
m.p.166.5-167.4°C (hexane/ethyl acetate) ¹H NMR (400MHz, CDCl₃) δ ; 2.46 (3H, s), 2.69 (3H, s), 7.36-7.42 (2H, m), 7.45-7.57 (3H, m), 7.96 (1H, s).

### Example 6 (Reference Example):

### Preparation of 5-acetyl-4-methyl-2-oxo-1-phenyl-1,2-di- hydro-pyridine-3-carbonitrile (Compound 71)

To a solution of the compound 70 (1.84g, 6.0mmol) prepared in Example 5 in N,N-dimethyl-formamide (15mL), there was added copper(I) cyanide (645mg, 7.2mmol) and then the resulting mixture was heated at 165°C for 5 hours with stirring. After the temperature of the mixture was brought back to room temperature, there were added, to the mixture, 28% aqueous ammonia (100mL) and sodium cyanide (2.94g, 60mmol) and the resulting mixture was further stirred for additional one hour. The reaction mixture was extracted twice with chloroform. The organic phases thus obtained were combined, washed with a saturated common salt aqueous solution, dried over anhydrous sodium sulfate and the solvent of the organic phase was distilled off. The residue thus recovered was purified by the silica gel column chromatography (hexane: ethyl acetate = 2:1 to 1:4) to thus give the title compound 71 (reference compound) (86.5mg, 57.7%) as a white powdery product.
m.p.215.4-216.8°C (hexane/ethyl acetate); ¹H NMR (400MHz, CDCl₃)^{™}; 2.48 (3H, s), 2.81 (3H, s), 7.36-7.41 (2H, m), 7.51-7.59 (3H, m), 8.16 (1H, s)

### Example 7:

### Preparation of 5-Acetyl-4-methyl-1-phenyl-3-(pyrimidin-2-yl-thio)-1H-pyridin- 2-one (Compound 74) (Preparation Method B; Step 4):

To a 60% sodium hydride solution (53.0g, 1.32mmol), there were added N,N-dimethylformamide (5.1 mL) and further 2-mercapto-pyrimidine (corresponding to the compound (VII); 171mg, 1.53mmol) and the resulting mixture was stirred at room temperature for 20 minutes. The reaction system was used in the subsequent step without any further treatment.

More specifically, to a solution of the compound 70 (312mg, 1.02mmol) prepared in Example 5 in N,N-dimethylformamide (5.1mL), there was added tetrakis(triphenylphosphine) palladium(0) (11.8mg, 0.0102mmol) and the resulting mixture was heated with stirring at 140°C for 20 minutes. The 2-mercapto-pyrimidine reagent prepared above was added to the foregoing mixture and the latter was further heated with stirring at 140°C for 1.5 hours. After the temperature of the mixture was brought back to room temperature, water was added thereto, the mixture was extracted with ethyl acetate, the resulting organic phases were combined together, washed with a saturated sodium chloride solution, dried over anhydrous sodium sulfate and then the solvent of the organic phase was removed through distillation. The residue thus recovered was purified by the silica gel column chromatography (hexane: ethyl acetate = 2:1 to 1:2) to thus give the title compound 74 (113mg, 33.0%). The product was further recrystallized from hexane/ethyl acetate to thus obtain the title compound 74 as yellow plate-like crystals.
m.p. 184.5-185.3°C; ¹H NMR (400MHz, CDCl₃) δ ; 2.49 (3H, s), 2.81 (3H, s), 6.96 (1H, t, J=4.7Hz), 7.39-7.54 (5H, m), 8.12 (1H, s), 8.46 (2H, d, J=4.7Hz)

The compounds (the compounds of the present invention and reference compounds) prepared by the same procedures used in the foregoing Examples 1 to 4 are summarized in the following Table 3. In this respect, various symbols appearing in the following Table 3 are the same as those specified above in connection with Table 1:

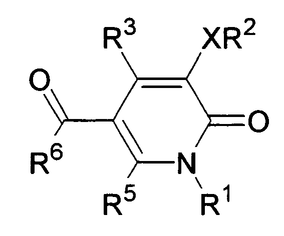

**Table 3**

| Compound No. | R¹ | X | R² | R³ | R⁶ | R⁵ |
|---|---|---|---|---|---|---|
| 70 (Reference) | Ph | - | Br | Me | Me | H |
| 71 (Reference) | Ph | - | CN | Me | Me | H |
| 72 | Ph | S | Bn | Me | Me | H |
| 73 | Ph | S | 4-Cl-Ph | Me | Me | H |
| 74 | Ph | S | 2-pyrimidinyl | Me | Me | H |
| 75 | Ph | S | Et | Me | Me | H |
| 76 | Ph | S | 4-OMe-Ph | Me | Me | H |

The results of ¹H NMR spectroscopic measurements of the foregoing compounds 70 to 76 are listed in the following Table 4.

**Table 4**

| Compound No | ¹H NMR {CDCl₃/TMS, δ (ppm)} |
|---|---|
| 72 | 2.40 (3H, s), 2.55 (3H, s), 4.20 (2H, s), 7.16-7.28 (5H, m), 7.3 1-7.45 (2H, m), 7.50-7.56 (3H, m), 7.90 (1H, s). |
| 73 | 2.47 (3H, s), 2.81 (3H, s), 7.15-7.22 (4H, m), 7.33-7.37 (2H, m ), 7.42-7.52 (3H, m), 8.06 (1H, s). |
| 75 | 1.20 (3H, t, J=7.5Hz), 2.45 (3H, s), 2.76 (3H, s), 3.00 (2H, q, J=7.5Hz), 7.37-7.55 (5H, m), 7.96 (1H, s). |
| 76 | 2.46 (3H, s), 2.84 (3H, s), 3.75 (3H, s), 6.75-6.80 (2H, m), 7.3 1-7.37 (4H, m), 7.41-7.52 (3H, m), 7.99 (1H, s). |

### Example 9:

### 3-(4-Fluoro-phenylthio)-5-(1-hydroxyethyl)-4-methyl-1-phenyl-1H-pyridin- 2-one (Compound 77) (Preparation Method D)

To a solution of the compound 1 (373mg, 1.06mmol) prepared in Example 1(b) in methanol (10.6mL), there was added sodium boron hydride (40.1mg, 1.06m). After stirring the mixture at room temperature for 10 minutes, a saturated ammonium chloride aqueous solution was added to the mixture and then the resulting mixture was extracted twice with ethyl acetate. The organic phases thus obtained were combined together, washed with a saturated sodium chloride solution, dried over anhydrous sodium sulfate and then the organic solvent was distilled off to thus give a residue. The residue thus recovered was purified by the silica gel column chromatography (hexane: ethyl acetate = 2:1 to 1:1) to thus give the title compound 77 (282mg, 75.2%) as a pale yellow syrup.
¹H NMR (400MHz, CDCl₃) δ ; 1.50 (3H, d, J=6.5Hz), 1.86 (1H, br), 2.59 (3H, s), 4.97 (1H, brq, J=6.5Hz), 6.88-6.94 (2H, m), 7.25-7.31 (3H, m), 7.32-7.41 (2H, m), 7.42-7.47 (2H, m), 7.55 (1H, s)

The compounds of the present invention prepared by the same procedures used in the foregoing Example are summarized in the following Table 5. In this respect, various symbols appearing in the following Table 5 are the same as those specified above in connection with Table 1.

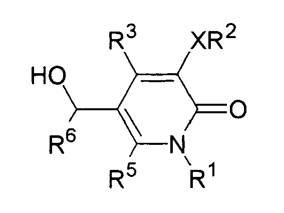

**Table 5**

| Compound No. | R¹ | X | R² | R³ | R⁶ | R⁵ |
|---|---|---|---|---|---|---|
| 77 | Ph | S | 4-F-Ph | Me | Me | H |
| 78 | 2-Cl-Ph | S | 4-F-Ph | Me | Me | H |
| 79 | Ph | S | 4-F-Ph | Me | n-Bu | H |

The results of ¹H NMR spectroscopic measurements of the foregoing compounds are listed in the following Table 6.

**Table 6**

| Compound No | ¹H NMR {CDCl₃/TMS, δ (ppm)} |
|---|---|
| 78 | 1.43 (3H, dd, J=1.4, 6.8Hz), 2.25-2.60 (1H, br), 2.55 (3H, d, J=1 .4Hz), 4.89 (1H, brquint, J=6.8Hz), 6.86-6.93 (2H, m), 7.20-7.38 (6H, m), 7.46-7.51 (1H, m). |
| 79 | 0.93 (3H, t, J=7.2Hz), 1.31-1.60 (4H, m), 1.62-1.80 (2H, m), 1.8 4 (1H, brd, J=4.0Hz), 2.57 (3H, s), 4.75 (1H, quint, J=4.0Hz), 6. 88-6.94 (2H, m), 7.26-7.30 (2H, m), 7.33-7.40 (3H, m), 7.42-7.47 (2H, m), 7.53 (1H, s). |

### Example 10: Preparation of 5-Ethyl-3-(4-fluoro-phenylthio)-4-methyl-1-phenyl-1H-pyridin-2-one (Compound 80) (Preparation Method E):

To powdery zinc (654mg, 10mmol), there was added a 0.5N hydrochloric acid solution (15mL) and the resulting mixture was stirred for 5 minutes. The mixture was washed twice with distilled water through the decantation operations. Distilled water was again added to the mixture, copper(II) sulfate penta-hydrate (12.5mg, 0.05mmol) was further added thereto and the mixture was stirred for 10 minutes. Again, the mixture was subjected twice (distilled water×2) to decantation treatments and the resulting product was used in the subsequent reaction step without subjecting the same to drying operations.

To a solution of the compound 1 (177mg, 0.5mmol) prepared in Example 1(b) in acetic acid (5mL), there were added the reagent prepared above and a 1N hydrochloric acid solution (5mL). After stirring the resulting mixture at room temperature for one hour, there were further added, to the mixture, powdery zinc (654mg, 10mmol) and a 1N hydrochloric acid solution (5mL). After stirring the mixture with heating at 80°C for one hour, the temperature thereof was brought back to room temperature, followed by the removal of the zinc through filtration, the addition of water to the filtrate and the subsequent extraction of the mixture with a hexane-ethyl acetate mixed solvent. The organic phases thus obtained were combined together, washed with a saturated sodium chloride solution, dried over anhydrous sodium sulfate and then the organic solvent was distilled off to thus give a residue. The residue thus recovered was purified by the silica gel column chromatography (hexane: ethyl acetate = 4:1) to thus give the title compound 80 (125mg, 73.5%) as a white powdery product.
m.p.116.8-118.7°C (hexane/ethyl acetate); ¹H NMR (400MHz, CDCl₃) δ ; 1.20 (3H, t, J=7.2Hz), 2.50 (2H, q, J=7.2Hz), 2.54 (3H, s), 6.88-6.95 (2H, m), 7.16 (1H, s), 7.25-7.48 (7H, m).

The compounds of the present invention prepared by the same procedures used in the foregoing Example are summarized in the following Table 7. In this respect, various symbols appearing in the following Table 7 are the same as those specified above in connection with Table 1:

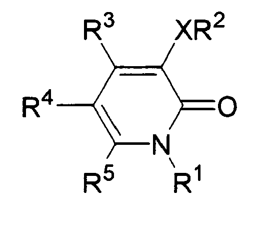

**Table 7**

| Compound No | R¹ | X | R² | R³ | R⁴ | R⁵ |
|---|---|---|---|---|---|---|
| 80 | Ph | S | 4-F-Ph | Me | Et | H |
| 81 | Ph | S | 4-F-Ph | Me | n-Pnt | H |

The results of ¹H NMR spectroscopic measurements of the foregoing compound 81 are listed below:
¹H NMR (400MHz, CDCl₃) δ ; 0.91 (3H, brt, J=6.8Hz), 1.33-1.39 (4H, m), 1.50-1.58 (2H, m), 2.44 (2H, dd, J=8.0, 8.6Hz), 2.53 (3H, s), 6.88-6.94 (2H, m), 7.17 (1H, s), 7.25-7.30 (2H, m), 7.34-7.39 (3H, m), 7.42-7.47 (2H, m).

### Example 11: Preparation of 3-(4-Methoxy-phenylthio)-4,5,6-trimethyl-1-phenyl-1H-pyridin-2-one (Compound 82) (Preparation Method F):

### (a) Step 1: Preparation of 3-methyl-4-phenylamino-3-penten-2-one (Compound (d); corresponding to the compound (XI))

3-Methyl-4-phenylimino-3-buten-2-one (the compound corresponding to the compound (IX) (see, Tetrahedron Lett., 1973, 52:5167)) (2.15g, 12.4mmol) was dissolved in diethyl ether (62mL) and then methyl magnesium bromide (the compound corresponding to the compound (X); 1.0M THF solution, 12.4mL, 12.4mmol) was added to the solution while stirring the solution under ice-cooling. After stirring the resulting mixture at room temperature for one hour, then a saturated aqueous solution of ammonium chloride solution was added thereto and the resulting mixture was extracted twice with diethyl ether. The organic phases thus obtained were combined together, washed with a saturated sodium chloride solution, dried over anhydrous sodium sulfate and then the organic solvent was distilled off to thus give a residue. The residue thus recovered was purified by the silica gel column chromatography (hexane: diethyl ether = 2:1) to thus give the title compound (d) (1.63g, 69.4%) as a pale yellow syrup.
¹H NMR (400MHz, CDCl₃) δ ; 1.92 (3H, s), 2.02 (3H, s), 2.22 (3H, s), 7.03-7.08 (2H, m), 7.14-7.19 (1H, m), 7.30-7.35 (2H, m)

### (b-1) Step 2: Preparation of N-(1,2-dimethyl-3-oxo-1-butenyl)-2-(4-methoxyphenyl-thio)-N-phenyl-acetamide) (Compound (e); corresponding to the compound (XII))

To a solution of the compound (d) (690mg, 3.65mmol) prepared above in tetrahydrofuran (36.5mL), there was added methyl magnesium bromide as a base (1.0M THF solution, 4.01mL, 4.01mmol) with stirring under ice-cooling. After 10 minutes, (4-methoxy-phenylthio)-acetyl chloride (corresponding to the compound (V); 1.03g, 4.75mmol) was then added to the foregoing mixture. After bringing the temperature of the mixture back to room temperature and stirring the mixture for 15 hours, water was added to the mixture and then the latter was stirred for 10 minutes. The reaction mixture was extracted twice with ethyl acetate, the organic phases thus obtained were combined together, washed with a saturated sodium chloride solution, dried over anhydrous sodium sulfate and then the organic solvent was distilled off to thus give a residue. The residue thus recovered was purified by the silica gel column chromatography (hexane: ethyl acetate = 2:1) to thus give the title compound (e) (375mg, 27.8%) as a paste-like product.
¹H NMR (400MHz, CDCl₃) δ ; 1.80-2.00 (6H, br), 2.30-2.40 (3H, br), 3.50-3.70 (2H, br), 3.70-3.85 (3H, br), 6.75-6.95 (2H, br), 7.10-7.60 (7H, br).

### (b-2) Steps 1 to 2: Preparation of N-(1,2-dimethyl-3-oxo-1-butenyl)-2-(4-methoxy-phenylthio)-N-phenyl-acetamide) (Compound (e); corresponding to the compound (XII)) (the method for preparing the title compound by a continuous steps while omitting the isolation step of the Compound (XI))

There was dissolved, in diethyl ether (20mL), 3-methyl-4-phenylimino-3-buten- 2-one (corresponding to the compound (IX); 693mg, 4.00mmol) and then methyl magnesium bromide (corresponding to the compound (X); a 10M THF solution, 4.40mL, 4.40mmol) was added to the solution with stirring under ice-cooling. After stirring at room temperature for one hour, there was added, to the resulting mixture, (4-methoxy-phenylthio)-acetyl chloride (corresponding to the compound (V); 1.13g, 5.2mM). After the temperature of the mixture was brought back to room temperature and the mixture was stirred over 15 hours, water was then added to the mixture, and the resulting mixture was stirred for additional 10 minutes. The reaction mixture was extracted twice with ethyl acetate, the organic phases were combined together, washed with a saturated sodium chloride solution, dried over anhydrous sodium sulfate, and then the solvent was removed through distillation. The residue thus recovered was purified by the silica gel column chromatography (hexane: ethyl acetate = 2:1) to thus give the title compound (e) (249mg, 16.8%) as a paste-like product.

### (c) Step 3: 3-(4-Methoxy-phenylthio)-4,5,6-trimethyl-1-phenyl-1H-pyridin-2-one (Compound 82; corresponding to the compound (I-i)):

To a solution of the compound (e) prepared above (624mg, 1.69mmol) in tetrahydrofuran (17mL), there was added 1,8-diazabicyclo[5.4.0]-7-undecene (1.26mg, 8.45mmol) with stirring under ice-cooling. After stirring the solution at room temperature for 14 hours, water was added to the solution followed by stirring the mixture for 10 minutes. The reaction mixture was extracted twice with ethyl acetate, the organic phases were combined together, washed with a saturated sodium chloride solution, dried over anhydrous sodium sulfate, and then the solvent was removed through distillation. The residue thus recovered was purified by the silica gel column chromatography (hexane: ethyl acetate = 1:1) to thus give the title compound 82 (284mg, 47.8%) as a paste-like product.
¹H NMR (400MHz, CDCl₃) δ ; 1.96 (3H, s), 2.11 (3H, s), 2.57 (3H, s), 3.75 (3H, s), 6.73-6.78 (2H, m), 7.10-7.14 (2H, m), 7.27-7.33 (2H, m), 7.36-7.41 (1H, m), 7.43-7.48 (2H, m).

The compounds of the present invention prepared by the same procedures used in the foregoing Example are summarized in the following Table 8. In this respect, various symbols appearing in the following Table 8 are the same as those specified above in connection with Table 1.

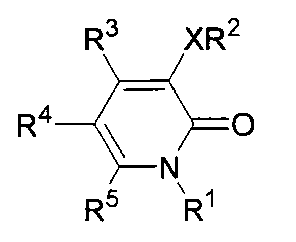

**Table 8**

| Compound No | R¹ | X | R² | R³ | R⁴ | R⁵ |
|---|---|---|---|---|---|---|
| 82 | Ph | S | 4-OMe-Ph | Me | Me | Me |
| 83 | Ph | S | 4-OMe-Ph | Me | Me | Et |

The results of ¹H NMR spectroscopic measurements of the foregoing compound 83 are listed below.
¹H NMR (400MHz, CDCl₃) δ ; 0.96 (3H, t, J=7.6Hz), 2.12 (3H, s), 2.36 (2H, q, J=7.6Hz), 2.57 (3H, s), 3.75 (3H, s), 6.74-6.78 (2H, m), 7.13-7.16 (2H, m), 7.28-7.33(2H, m), 7.37-7.49 (3H, m)

### Examples of Pharmaceutical Preparations

**Tablet: Tablets were prepared, which had a composition specified below, a diameter of 9mm and a weight of 200mg.**

| Component | Amt. (g) |
|---|---|
| Compound 69 | 1.0 |
| Hydroxypropyl cellulose | 0.8 |
| Silicon dioxide | 0.2 |
| Crystalline cellulose | 0.5 |
| Talc | 0.5 |

**Capsules (Preparation packed in a hard capsule): There were prepared encapsulated pharmaceutical preparations packed in hard capsules, having a composition specified below:**

| Component | Amt. (g) |
|---|---|
| Compound 69 | 1.0 |
| Crystalline cellulose | 1.0 |
| Lactose | 1.5 |
| Silicon dioxide | 0.2 |

**Granules: There were prepared granules having a composition specified below:**

| Component | Amt. (g) |
|---|---|
| Compound 69 | 2.0 |
| Lactose | 2.0 |
| Hydroxypropyl cellulose | 3.0 |
| Talc | 0.2 |

**Cream: There were prepared a cream having a composition specified below:**

| Component | Amt. (% by mass) |
|---|---|
| Compound 69 | 1.0 |
| Cholesterol | 0.5 |
| Cholesteryl isostearate | 1.0 |
| Polyether-modified silicone | 1.5 |
| Cyclic silicone | 15 |
| Methylphenyl polysiloxane | 2.0 |
| Methyl polysiloxane | 2.0 |
| Magnesium sulfate | 0.5 |
| 50% Ethanol solution | 5.0 |
| Carboxymethyl chitin | 0.5 |
| Purified water | Balance |
| Sum | 100.0 |

### Methods for Inspecting Compounds for Cell Adhesion-Inhibitory Effects and Effects on Neutrophil-Infiltration:

The inspection of candidate compounds for the cell adhesion-inhibitory effect and the effect on the neutrophil-infiltration can be carried out while taking into consideration the methods disclosed in the literature. For instance, the following articles disclose the methods for testing the activities of candidate compounds: Carbohydrate Letters, Vol. 1, pp. 261-268; and J. Pharm. Exp. Therapeutics, Vol. 269, No. 3, p. 917.

### Test Example 1: Test for Examining Cell Adhesion-Inhibitory Effect

To a 96-well culture plate coated, in advance, with keyhole limpet hemocyanin, there was added 0.05mL each of a solution of a candidate test compound in dimethylsulfoxide diluted to a final dimethylsulfoxide concentration of 1% with DMEM. A blood sample was collected from a healthy person through the median vein of elbow and the neutrophils were fractionated or isolated through the use of Mono-Poly Separation Liquid and the centrifugation. The fractionated neutrophils were suspended in DMEM to a concentration of 1 × 10⁶ cells/mL and 0.1mL of the resulting neutrophil-containing suspension was added to each well of the culture plate. Moreover, 40 ng/mL of tumor-necrosis factor (TNF)-α was diluted with DMEM to a final concentration of 10ng/mL and 0.05mL of the resulting diluted solution was added to each well of the culture plate. After the culture plate was allowed to stand at 37°C for 30 minutes in a CO₂-incubator, the plate was washed three times (thrice) with 0.2mL of phosphate-buffered physiological saline (PBS(-), available from GIBCO Corporation). In addition, to each well, there was added 0.1mL of a 10% solution of Alamar blue in DMEM, the culture plate was allowed to stand at 37°C for 3 hours in a CO₂-incubator and then the intensity of fluorescent rays emitted from the adhered neutrophil cells was determined (Ex: 544nm; Em: 590nm). In this respect, the sample group free of any stimulation was defined to be the non-adhesion control group and the sample group stimulated with TNF-α was referred to as the adhesion control group to thus determine or calculate the cell adhesion-inhibitory rate of the compound of the present invention at each concentration, and the 50%-inhibitory concentration (IC₅₀) was calculated from the cell adhesion-inhibitory rates thus determined according to the Probit technique. The same test procedures used above were repeated except for using different kinds of candidate compounds.

The results obtained in the foregoing tests are listed in the following Table 9:

**Table 9**

| Compound No. | IC₅₀ (µM) |
|---|---|
| 1 | 0.400 |
| 20 (Reference) | 2.92 |
| 55 (Reference) | 9.52 |
| 56 | 0.590 |
| 62 | 0.569 |
| 66 | 0.049 |
| 67 | 0.053 |
| 68 | 0.287 |
| 69 | 0.051 |
| 76 | 0.450 |
| 77 | 0.980 |

The results listed in the foregoing Table 9 clearly indicate that the compounds of the present invention wherein the substituent X in the general formula represents a sulfur (S) atom were quite excellent in the IC₅₀ (µM) values as compared with those observed for the compounds (such as Compounds 20 and 55) in which the substituent X in the general formula represents a carbon or oxygen atom.

### Test Example 2: Effect on Zymosan-Induced Neutrophil Infiltration

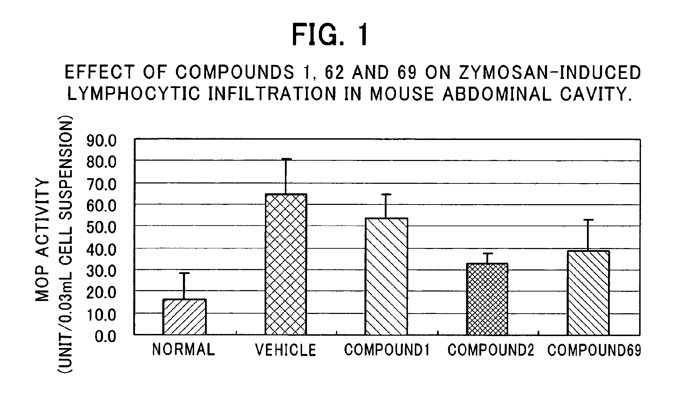

Each candidate compound was administered to 6-week-old male ICR mice, which had been fasted from the day before the initiation of this test. Each candidate compound was suspended in a 3% dimethylsulfoxide/carboxymethyl cellulose sodium solution and the resulting suspension was administered to the mice at a dose of 30mg/kg body weight/10mL. After the elapse of one hour, 1mL of a 0.01% solution of zymosan in physiological saline was intra-peritoneally administered. After 3 hours, 5mL of physiological saline was intra-peritoneally administered and the test animals were lightly massaged for 30 seconds. The abdominal wall of the test animals were slightly incised to thus collect the peritoneal fluid using a Pasteur pipette. Thereafter, the fluid thus recovered was centrifuged (at 20,000g, for 20 minutes, at 4°C), the resulting pellets were washed with a 50mM sodium phosphate buffer (pH 6.0) and then again centrifuged. To the pellets thus recovered, there was added 0.5% hexadecyl trimethyl ammonium bromide (HTAB) and then centrifuged to thus give a supernatant, which was subsequently used as an enzyme source. To 15 µL of the enzyme source, there were added 200 *µ*L of a 1.154mM o-di-anisidine and 50 µL of a 0.005% hydrogen peroxide solution and then the optical densities at 450nm (OD₄₅₀) were determined to thus calculate the difference: (OD₄₅₀ observed after 90 seconds from the initiation of the reaction) - (OD₄₅₀ observed after 30 seconds from the initiation of the reaction), which was herein defined to be MPO activity. In this connection, the MPO activity was calculated, while defining the activity required for increasing the OD450 value by 0.001 as one unit. The MPO activity is herein defined to be the activity of the enzyme present in the neutrophils and accordingly, the reduction in the MPO activity means the inhibition of the neutrophil-infiltration. The results thus obtained are plotted on Fig. 1.

In Fig. 1, the term "normal" means the case free of any zymosan-induction (in case where only physiological saline was intra-peritoneally administered to test animals) and the term "vehicle" means the case in which the solvent free of any candidate compound was orally administered to test animals, prior to the zymosan- induction.

The results plotted on Fig. 1 clearly indicate that the compound of the present invention shows the desired neutrophil infiltration-inhibitory effect. More specifically, the animal groups, to which Compound 1, Compound 62 and Compound 69 were administered, respectively, there were observed decreases in the MPO activity with respect to the "vehicle". Accordingly, this indicates that the neutrophil infiltration is surely inhibited. In particular, it would be concluded that Compound 62 and Compound 69 show excellent neutrophil infiltration-inhibitory effects.

### Test Example 3: Toxicity Test by Administration of One time (Single Dose)

### Test Animals Used: ICR Male Mice (5-week-old):

They were purchased and then preliminarily kept over one week, prior to the practical use thereof in this test.

Each test group comprised 5 animals.
Inspected Items: Appearance (such as death, weakening); Physical Conditions (such as the presence of lateral turning); Consciousness (such as excitation, lethargy (coma)); Behaviors (such as convulsions); Properties of fecal matters; and Foreign matters present in the urine.

| Compound Administered | Amt. of Single Dose | Result |
|---|---|---|
| Compound 1 | 1g/kg | All of the test animals were not died and there was not observed any severe side-effect. |
| Compound 69 | 2g/kg | All of the test animals were not died and there was not observed any severe side-effect. |

### (Effect of the Invention)

The foregoing experimental results clearly indicate that the compound of the present invention possesses an intensive activity to inhibit any cell adhesion between the lymphocytes and the vascular endothelial cells and shows a neutrophil infiltration-inhibitory effect. Moreover, the results observed in the single dose test likewise clearly indicate that the compound of the present invention has extremely low toxicity. Accordingly, the compound and pharmaceutical composition containing the same according to the present invention are useful for preventing and/or treating the diseases associated with the adhesion of leukocytes to a variety of cells, for instance, inflammatory diseases, allergies, and immune diseases; and likewise effective for preventing and/or treating the disease conditions associated with the lymphocytic infiltration, such as rheumatism, metastasis of cancer, asthmas, diabetes, autoimmune diseases. For instance, the compound of the present invention is effective for preventing and/or treating the inflammatory dermatitis such as atopic dermatitis, psoriasis, contact dermatitis and eczema; wounds; burn injuries; infiltration/metastasis of neoplastic or carcinomatous tumors; rejections against transplantation; asthmas; chronic rheumatoid arthritis; arteriosclerosis; acute respiratory distress syndrome (ADRS); ischemia reperfusion; ulcerative colitis; Crohn disease; multiple sclerosis; systemic lupus erythemathosus (SLE); and osteoporosis.

### Industrial Applicability

According to the present invention, there is thus provided a 2-pyridone derivative which possesses an intensive cell adhesion-inhibitory effect, can control the lymphocytic infiltration action, which has an excellent anti-inflammatory effect, an anti-asthmatic effect, an anti-rheumatic effect, an anti-arteriosclerotic effect, an anti-allergic effect, an effect of controlling metastasis of tumors, an effect of inflammatory disorders accompanied with operations and/or treatments, an effect of controlling non-occlusive mesenteric infarction, an effect of controlling any rejections against transplantation, an anti-psoriatic effect, and anti-ADRS (acute respiratory distress syndrome) effect and which is accordingly, useful for treating inflammatory intestinal diseases and burn injuries.

### Brief Description of the Drawing

Fig. 1 is a diagram showing the effect of controlling neutrophil infiltration observed in a zymosan-induced model.

## Claims

1. A 2-pyridone derivative represented by the following formula (1) or a pharmaceutically acceptable salt thereof: 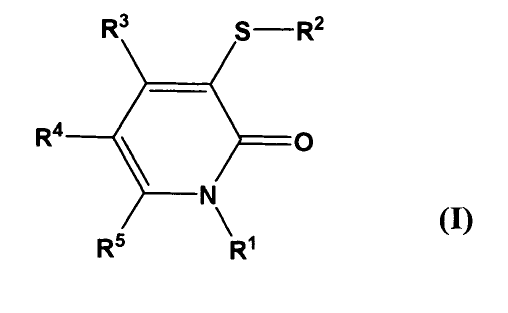 wherein R¹ represents a hydrogen atom, a lower alkyl group, a cycloalkyl group, an aryl group or an aromatic heterocyclic group;
R² represents a hydrogen atom, a halogen atom, a cyano group, a lower alkyl group, an aryl group or an aromatic heterocyclic group;
R³ represents a lower alkyl group or an aryl group;
R⁴ represents a lower alkyl group, an aryl group, -C(=O)-R⁶, or -CH(OH)-R⁶ (wherein R⁶ represents a lower alkyl group or an aryl group); and
R⁵ represents a hydrogen atom, a lower alkyl group or an aryl group.

2. A pharmaceutical composition comprising, as an effective component, a 2-pyridone derivative or a pharmaceutically acceptable prodrug or salt thereof as set forth in claim 1.

3. The pharmaceutical composition as set forth in claim 2, wherein the composition is used for preventing and/or treating disease conditions associated with lymphocytic infiltration.

4. A method for preparing a 2-pyridone derivative represented by the following formula (1): 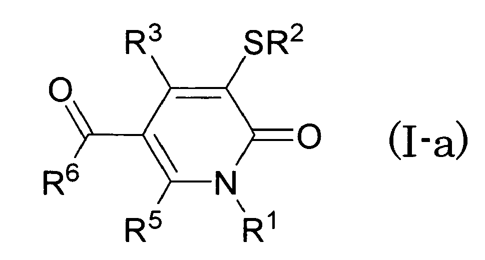 wherein R¹ represents a hydrogen atom, a lower alkyl group, a cycloalkyl group, an aryl group or an aromatic heterocyclic group;
R² represents a hydrogen atom, a halogen atom, a cyano group, a lower alkyl group, an aryl group or an aromatic heterocyclic group;
R³ represents a lower alkyl group or an aryl group;
R⁵ represents a hydrogen atom, a lower alkyl group or an aryl group); and
R⁶ represents a lower alkyl group or an aryl group,
said method comprising reacting a compound (IV) represented by the following formula (IV): 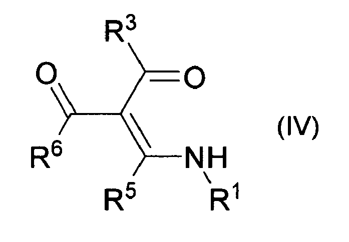 wherein R¹, R³, R⁵, and R⁶ are the same as those specified above, with a compound (V) represented by the following formula (V): 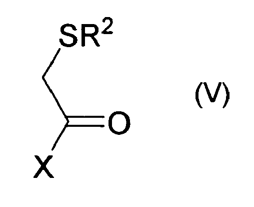 wherein R² is the same as that specified above, and X represents a halogen atom.

5. A method for preparing a 2-pyridone derivative represented by the following formula (1): 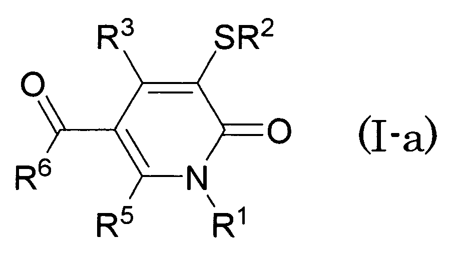 wherein R¹ represents a hydrogen atom, a lower alkyl group, a cycloalkyl group, an aryl group or an aromatic heterocyclic group;
R² represents a hydrogen atom, a halogen atom, a cyano group, a lower alkyl group, an aryl group or an aromatic heterocyclic group;
R³ represents a lower alkyl group or an aryl group;
R⁵ represents a hydrogen atom, a lower alkyl group or an aryl group); and
R⁶ represents a lower alkyl group or an aryl group,
said method comprising the steps of:
(1) reacting a compound (IV) represented by the following formula (IV): 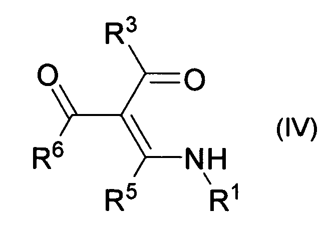 wherein R¹, R³, R⁵, and R⁶ are the same as those specified above) with a compound (VI) represented by the following formula (VI): 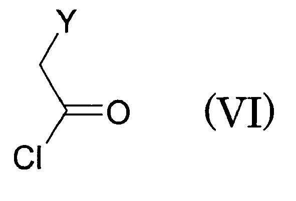 wherein Y represents a halogen atom, to thus form a compound (I-b) represented by the following formula (I-b): 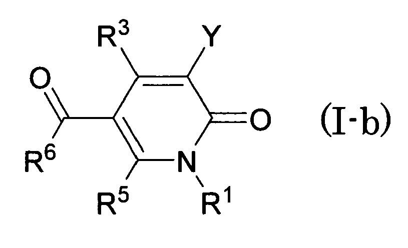 wherein R¹, R³, R⁵, R⁶ and Y are the same as those specified above; and then
(2) reacting the resulting compound (I-b) with HSR² (wherein, R² is the same as that specified above).

6. A method for preparing a compound represented by the following formula (1): 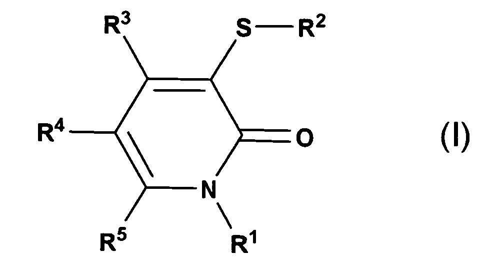 wherein R¹ represents a hydrogen atom, a lower alkyl group, a cycloalkyl group, an aryl group or an aromatic heterocyclic group;
R² represents a hydrogen atom, a halogen atom, a cyano group, a lower alkyl group, an aryl group or an aromatic heterocyclic group;
R³ represents a lower alkyl group or an aryl group;
R⁴ represents a lower alkyl group, an aryl group, -C(=O)-R⁶, or -CH(OH)-R⁶ (wherein R⁶ represents a lower alkyl group or an aryl group); and
R⁵ represents a hydrogen atom, a lower alkyl group or an aryl group,
said method comprising the steps of:
(1) preparing a compound (XII) represented by the following formula (XII): 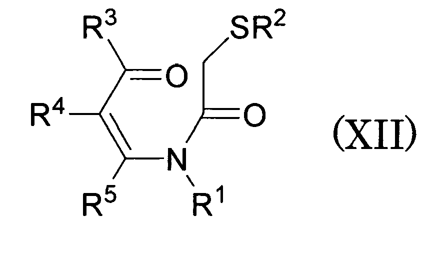 wherein R¹ to R⁵ are the same as those specified above, and then
(2) treating the resulting compound (XII) with a base.
